# EUROPEAN PATENT APPLICATION

(11) **EP 4 386 010 A1**
(43) Date of publication of application: **19.06.2024**
(21) Application number: 22855494.5
(22) Date of filing: 11.08.2022
(51) Int. Cl.: C07K 19/00, C07K 14/74, C12N 15/62, C12N 5/0783, A61K 35/17, A61P 35/00

(54) **MODIFIED CELL, AND PREPARATION METHOD THEREFOR AND USE THEREOF**

(30) Priority: 12.08.2021 CN 202110925291
(71) Applicant: Cure Genetics Co., Ltd., Suzhou, Jiangsu 215123 (CN)
(72) Inventor: HUANG, Zhuo, Suzhou, Jiangsu 215123 (CN); LIN, Yanni, Suzhou, Jiangsu 215123 (CN); ZHENG, Xiaocui, Suzhou, Jiangsu 215123 (CN)
(74) Representative: Engelhard, Markus
(86) International application number: PCT/CN2022/111713
(87) International publication number: WO 2023/016514

(57) **Abstract**

A modified cell, a method for preparing a modified cell, and a use thereof in treatment of a disease. The modified cell comprises a polynucleotide that encodes a presenting peptide, wherein the polynucleotide is under the transcriptional regulation of an endogenous B2M gene.

## Description

### Technical Field

The present application relates to the field of biomedicine, specifically to a modified cell, a method for preparing the modified cell, and their use. The modified cell has reduced allogeneic immune response.

### Background Art

Cell therapy has good efficacy in major diseases such as various tumors (such as relapsed and refractory lymphoma, leukemia, multiple myeloma, *etc*.)*,* and has become a research and development hotspot in recent years. Immune cells in existing cell therapies are usually obtained from the patient's autologous cells, modified and then infused back into the same patient. There are problems of long production cycle and high preparation cost, and in many cases, the patient's cell is in a not good state, which makes it difficult to prepare modified cells successfully.

In order to solve the rejection problem of cell allogeneic transplantation, on the one hand, the expression of the TCR complex of the donor immune cells is removed to prevent the donor immune cells from recognizing and killing the host cells; on the other hand, the expression of MHC class I molecules in donor immune cells is removed to eliminate antigen presentation by donor cells so that the donor immune cells evade the recognition and killing by host immune cells. However, the deletion of MHC class I molecules may initiate receptor NK cell-mediated killing. Therefore, there is an urgent need for novel modified cells that can resist T cell and NK cell rejection at the same time, have a simple preparation process, and are suitable for industrial application.

### Summary of the Invention

The present application provides a modified cell and a method for preparing the cell. The present application realizes the insertion, replacement or correction of genomic sequences encoding restricted presentating peptides in living cells through gene editing technology based on the CRISPR/Cas system, especially through any known site-specific method, so that knock-out/down-regulation of specific MHC complexes is achieved, meanwhile another specific MHC complex is overexpressed, thereby preparing universal T cells that do not induce graft-versus-Host Disease (GvHD) and simultaneously resist host-versus-graft response (HvGR), especially resist allogeneic T cell and NK cell rejection.

In one aspect, the present application provides a modified cell comprising a polynucleotide that encodes a presenting peptide, wherein the polynucleotide is under the transcriptional regulation of an endogenous B2M gene.

In certain embodiments, the polynucleotide is in the locus of the endogenous B2M gene.

In certain embodiments, the modified cell is capable of expressing a B2M protein.

In certain embodiments, the presenting peptide is capable of covalently binding to the B2M protein.

In certain embodiments, at least a portion of the B2M protein is encoded by the endogenous B2M gene.

In certain embodiments, the modified cell is capable of expressing the B2M protein, and the B2M protein is substantially encoded by the endogenous B2M gene.

In certain embodiments, the expression and/or activity of the B2M protein on the cell surface is substantially not down-regulated compared with cells without the modification.

In certain embodiments, the expression and/or activity of the B2M protein on the cell surface is down-regulated by no more than about 80%, about 70%, about 60%, about 50%, about 40%, about 30%, about 20% or less compared with cells without the modification.

In certain embodiments, the polynucleotide further comprises a nucleic acid molecule encoding a fragment of the B2M protein.

In certain embodiments, the polynucleotide does not comprise a complete B2M gene coding region.

In certain embodiments, the polynucleotide does not comprise a nucleic acid molecule encoding a heavy chain of an MHC molecule.

In certain embodiments, the polynucleotide is in the same open reading frame as the endogenous B2M gene.

In certain embodiments, the polynucleotide is operably linked to a promoter of the endogenous B2M gene.

In certain embodiments, the polynucleotide is located between a nucleic acid molecule encoding a signal peptide and a nucleic acid molecule encoding a B2M mature protein.

In certain embodiments, the modified cells have reduced allogeneic rejection response compared to cells without the modification.

In certain embodiments, the presenting peptide is capable of forming a complex with the B2M protein and the heavy chain of the MHC molecule.

In certain embodiments, the complex is capable of inhibiting immune cell activation.

In certain embodiments, the immune cell is selected from the group comprising a natural killer (NK) cell, a T lymphocyte, a B lymphocyte, a NKT cell, a monocyte, a macrophage, a mast cell, a granulocyte, a dendritic cell, a lymphocyte, a leukocyte and/or a peripheral blood mononuclear cell.

In certain embodiments, the immune cell is a natural killer (NK) cell, a NKT cell and/or a T lymphocyte.

In certain embodiments, the inhibiting immune cell activation comprises inhibiting an immune cell from participating in an immune response.

In certain embodiments, the inhibiting immune cell activation comprises binding to an inhibitory receptor of the immune cell.

In certain embodiments, the inhibitory receptor of the immune cell is selected from the group comprising NKG2A, NKG2B, KIR2DL1, KIR2DL2, KIR2DL3, KIR2DL4, KIR2DL5, KIR3DL1, KIR3DL2 and KIR3DL3.

In certain embodiments, the heavy chain of the MHC molecule comprises a heavy chain of a non-classical MHC class I molecule and/or a heavy chain of an MHC-like molecule.

In certain embodiments, the heavy chain of the non-classical MHC class I molecule comprises an HLA-E heavy chain, an HLA-F heavy chain and/or an HLA-G heavy chain.

In certain embodiments, the heavy chain of the MHC-like molecule comprises a CD1 heavy chain, an MR1 heavy chain, an FcRn heavy chain and/or a UL18 heavy chain.

In certain embodiments, the heavy chain of the MHC molecule is an HLA-E heavy chain.

In certain embodiments, the presenting peptide is capable of binding to the peptide binding groove of the heavy chain of the MHC molecule.

In certain embodiments, the presenting peptide is derived from a virus, a prokaryote, an eukaryote or a mammal.

In certain embodiments, the presenting peptide is derived from a human.

In certain embodiments, the presenting peptide comprises a non-classical MHC class I restricted presenting peptide and/or an MHC-like molecule restricted presenting peptide.

In certain embodiments, the presenting peptide is a non-classical MHC class I restricted presenting peptide.

In certain embodiments, the presenting peptide is an HLA-E restricted presenting peptide.

In certain embodiments, the presenting peptide is derived from a protein selected from the group comprising an ATP-binding cassette transporter, a multidrug resistance-associated protein 7, gliadin, an HSP60 conserved region and a GroEL conserved region.

In certain embodiments, the presenting peptide is derived from a signal peptide of a MHC class I molecule, or a fragment thereof.

In certain embodiments, the presenting peptide is derived from a signal peptide of a protein selected from the group comprising HLA-Al, HLA-A2, HLA-A*02, HLA-A*0203, HLA-A*0210, HLA-A3, HLA-A*23, HLA-A*24, HLA-A*2403, HLA-A*2410, HLA-A*2502, HLA-A*2501, HLA-A*26, HLA-A*66(01,02), HLA-A9, HLA-A*6603, HLA-A10, HLA-A*11, HLA-A19, HLA-A*29, HLA-A*30, HLA-A*31, HLA-A*32, HLA-A*33, HLA-A*74, HLA-A*7403, HLA-A28, HLA-A36, HLA-A34, HLA-A43, HLA-A*3401, HLA-A*80, HLA-B5, HLA-B7, HLA-B8, HLA-B12, HLA-B13, HLA-B14, HLA-B15, HLA-B16, HLA-B17, HLA-B18, HLA-B21, HLA-B22, HLA-B27, HLA-B35, HLA-B37, HLA-B38, HLA-B39, HLA-B40, HLA-B41, HLA-B42, HLA-B46, HLA-B47, HLA-B48, HLA-B49, HLA-B50, HLA-B51, HLA-B52, HLA-B53, HLA-B54, HLA-B55, HLA-B56, HLA-B57, HLA-B58, HLA-B59, HLA-B60, HLA-B61, HLA-B62, HLA-B63, HLA-B64, HLA-B65, HLA-B67, HLA-B70, HLA-B71, HLA-B73, HLA-B75, HLA-B76, HLA-B77, HLA-B78, HLA-B81, HLA-B82, HLA-B83, HLA-Cw1, HLA-Cw2, HLA-Cw3, HLA-Cw4, HLA-Cw5, HLA-Cw6, HLA-Cw7, HLA-Cw8, HLA-Cw9, HLA-Cw10, HLA-Cw*0809, HLA-Cw7, HLA-Cw*1701, HLA-G and HLA-F.

In certain embodiments, the presenting peptide is 5-30 amino acids in length.

In certain embodiments, the presenting peptide is 7-20 amino acids in length.

In certain embodiments, the presenting peptide is 8-10 amino acids in length.

In certain embodiments, the presenting peptide comprises an amino acid sequence as shown in SEQ ID NO: 17.

In certain embodiments, the presenting peptide comprises an amino acid sequence as shown in any one of SEQ ID NOs: 18-70.

In certain embodiments, the presenting peptide comprises an amino acid sequence as shown in SEQ ID NO: 35, SEQ ID NO: 30 or SEQ ID NO: 33.

In certain embodiments, the polynucleotide further comprises a nucleic acid molecule encoding a linker.

In certain embodiments, the presenting peptide and the B2M protein are linked via the linker in an expression product of the modified cell.

In certain embodiments, the linker comprises a rigid linker and/or a flexible linker.

In certain embodiments, the linker comprises an amino acid sequence as shown below: (EAAAK)ₙ, where n is 3, 4, or 5.

In certain embodiments, the linker comprises an amino acid sequence as shown below: (GGGGS)ₙ, where n is 3, 4, or 5.

In certain embodiments, the linker comprises an amino acid sequence as shown in any one of SEQ ID NOs: 71-99.

In certain embodiments, the linker comprises an amino acid sequence as shown in SEQ ID NO: 99.

In certain embodiments, the linker is located at the C-end of the presenting peptide.

In certain embodiments, the polynucleotide does not comprise a nucleic acid molecule encoding a B2M protein or a fragment thereof.

In certain embodiments, the polynucleotide encodes an amino acid sequence as shown in SEQ ID NO: 9.

In certain embodiments, the polynucleotide comprises a nucleotide sequence as shown in SEQ ID NO: 12.

In certain embodiments, the modified cell is used for cell transplantation, tissue transplantation, and/or organ transplantation.

In certain embodiments, the modified cell comprises a mammalian cell.

In certain embodiments, the modified cell comprises a stem cell and/or a somatic cell.

In certain embodiments, the modified cell comprises a blood cell, a muscle cell, a nerve cell, and/or an immune cell.

In certain embodiments, the modified cell comprises an immune cell selected from the group comprising a natural killer (NK) cell, a T lymphocyte, a B lymphocyte, a NKT cell, a monocyte, a macrophage, a mast cell, a granulocyte, a dendritic cell, a lymphocyte, a leukocyte and/or a peripheral blood mononuclear cell.

In certain embodiments, the modified cell is a natural killer (NK) cell, a NKT cell and/or a T lymphocyte.

In certain embodiments, the expression of a classical MHC class I molecule on the cell surface of the modified cell is down-regulated, and the expression of a non-classical MHC class I molecule is positive.

In certain embodiments, the expression of a classical MHC class I molecule on the cell surface of the modified cell is down-regulated, and the expression of a non-classical MHC class I molecule is substantially not down-regulated.

In certain embodiments, the expression of a classical MHC class I molecule on the cell surface of the modified cell is down-regulated, and the expression of a non-classical MHC class I molecule is up-regulated.

In certain embodiments, the expression and/or activity of a T cell receptor α constant region protein (TRAC) of the modified cell is down-regulated. For example, the expression and/or activity of the T cell receptor α constant region protein (TRAC) of the modified cell is down-regulated by at least 20%, 20%, 40%, 50 %, 60%, 70%, 80%, 90% or more compared with cells without the modification.

In certain embodiments, the modified cell further comprises a chimeric antigen receptor (CAR), a T cell receptor (TCR), a chimeric autoantibody receptor (CAAR), a TCR receptor fusion construct (TRuC), a T cell antigen coupler (TAC), an antibody TCR receptor (AbTCR), a chimeric CD3ε receptor and/or at least one synthetic receptor.

On the other hand, the present application provides a method for preparing a modified cell, the method comprises: inserting a polynucleotide encoding a presenting peptide into the cell such that the polynucleotide is under the transcriptional regulation of an endogenous B2M gene.

In certain embodiments, the modified cell is capable of expressing a B2M protein.

In certain embodiments, the presenting peptide is capable of covalently binding to the B2M protein.

In certain embodiments, the modified cell is capable of expressing a B2M protein, and at least a portion of the B2M protein is encoded by the endogenous B2M gene

In certain embodiments, the modified cell is capable of expressing the B2M protein, and the B2M protein is substantially encoded by the endogenous B2M gene.

In certain embodiments, the inserting does not substantially down-regulate the expression and/or activity of the B2M protein on the surface of the modified cell compared with cells without the modification.

In certain embodiments, the inserting reduces the expression and/or activity of the B2M protein on the surface of the modified cell by no more than 80%, about 70%, about 60%, about 50% %, about 40%, about 30%, about 20% or less compared with cells without the modification.

In certain embodiments, the polynucleotide further comprises a nucleic acid molecule encoding a fragment of the B2M protein.

In certain embodiments, the polynucleotide does not comprise a complete B2M gene coding region.

In certain embodiments, the polynucleotide does not comprise a nucleic acid molecule encoding a heavy chain of an MHC molecule.

In certain embodiments, the inserting comprises inserting the polynucleotide into the locus of the endogenous B2M gene.

In certain embodiments, the inserting comprises inserting the polynucleotide into the same open reading frame as the endogenous B2M gene.

In certain embodiments, the inserting operably links the polynucleotide to a promoter of the endogenous B2M gene.

In certain embodiments, the inserting comprises inserting the polynucleotide between a nucleic acid molecule encoding a signal peptide and a nucleic acid molecule encoding a B2M mature protein.

In certain embodiments, the modified cells have reduced allogeneic rejection response compared to cells without the modification.

In certain embodiments, the presenting peptide is capable of forming a complex with the B2M protein and the heavy chain of the MHC molecule.

In certain embodiments, the complex is capable of inhibiting immune cell activation.

In certain embodiments, the immune cell is selected from the group comprising a natural killer (NK) cell, a T lymphocyte, a B lymphocyte, a NKT cell, a monocyte, a macrophage, a mast cell, a granulocyte, a dendritic cell, a lymphocyte, a leukocyte and/or a peripheral blood mononuclear cell.

In certain embodiments, the immune cell is a natural killer (NK) cell, a NKT cell and/or a T lymphocyte.

In certain embodiments, the inhibiting immune cell activation comprises inhibiting an immune cell from participating in an immune response.

In certain embodiments, the inhibiting immune cell activation comprises binding to an inhibitory receptor of the immune cell.

In certain embodiments, the inhibitory receptor of the immune cell is selected from the group comprising NKG2A, NKG2B, KIR2DL1, KIR2DL2, KIR2DL3, KIR2DL4, KIR2DL5, KIR3DL1, KIR3DL2 and KIR3DL3.

In certain embodiments, the heavy chain of the MHC molecule comprises a heavy chain of a non-classical MHC class I molecule and/or a heavy chain of an MHC-like molecule.

In certain embodiments, the heavy chain of the non-classical MHC class I molecule comprises an HLA-E heavy chain, an HLA-F heavy chain and/or an HLA-G heavy chain.

In certain embodiments, the heavy chain of the MHC-like molecule comprises a CD1 heavy chain, an MR1 heavy chain, an FcRn heavy chain and/or a UL18 heavy chain.

In certain embodiments, the heavy chain of the MHC molecule is an HLA-E heavy chain.

In certain embodiments, the presenting peptide is capable of binding to the peptide binding groove of the heavy chain of the MHC molecule.

In certain embodiments, the presenting peptide is derived from a virus, a prokaryote, an eukaryote or a mammal.

In certain embodiments, the presenting peptide is derived from a human.

In certain embodiments, the presenting peptide comprises a non-classical MHC class I restricted presenting peptide and/or an MHC-like molecule restricted presenting peptide.

In certain embodiments, the presenting peptide is a non-classical MHC class I restricted presenting peptide.

In certain embodiments, the presenting peptide is an HLA-E restricted presenting peptide.

In certain embodiments, the presenting peptide is derived from a protein selected from the group comprising an ATP-binding cassette transporter, a multidrug resistance-associated protein 7, gliadin, an HSP60 conserved region and a GroEL conserved region.

In certain embodiments, the presenting peptide is derived from a signal peptide of a MHC class I molecule, or a fragment thereof.

In certain embodiments, the presenting peptide is derived from a signal peptide of a protein selected from the group comprising HLA-Al, HLA-A2, HLA-A*02, HLA-A*0203, HLA-A*0210, HLA-A3, HLA-A*23, HLA-A*24, HLA-A*2403, HLA-A*2410, HLA-A*2502, HLA-A*2501, HLA-A*26, HLA-A*66(01,02), HLA-A9, HLA-A*6603, HLA-A10, HLA-A*11, HLA-A19, HLA-A*29, HLA-A*30, HLA-A*31, HLA-A*32, HLA-A*33, HLA-A*74, HLA-A*7403, HLA-A28, HLA-A36, HLA-A34, HLA-A43, HLA-A*3401, HLA-A*80, HLA-B5, HLA-B7, HLA-B8, HLA-B12, HLA-B13, HLA-B14, HLA-B15, HLA-B16, HLA-B17, HLA-B18, HLA-B21, HLA-B22, HLA-B27, HLA-B35, HLA-B37, HLA-B38, HLA-B39, HLA-B40, HLA-B41, HLA-B42, HLA-B46, HLA-B47, HLA-B48, HLA-B49, HLA-B50, HLA-B51, HLA-B52, HLA-B53, HLA-B54, HLA-B55, HLA-B56, HLA-B57, HLA-B58, HLA-B59, HLA-B60, HLA-B61, HLA-B62, HLA-B63, HLA-B64, HLA-B65, HLA-B67, HLA-B70, HLA-B71, HLA-B73, HLA-B75, HLA-B76, HLA-B77, HLA-B78, HLA-B81, HLA-B82, HLA-B83, HLA-Cw1, HLA-Cw2, HLA-Cw3, HLA-Cw4, HLA-Cw5, HLA-Cw6, HLA-Cw7, HLA-Cw8, HLA-Cw9, HLA-Cw10, HLA-Cw*0809, HLA-Cw7, HLA-Cw*1701, HLA-G and HLA-F. For example, the above-mentioned proteins represent multiple types of HLA, and HLA typing can be performed through cytology (for example, flow cytometry), serology, and genomics (for example, first-generation sequencing, second-generation sequencing, and gene chip).

In certain embodiments, the presenting peptide is 5-30 amino acids in length.

In certain embodiments, the presenting peptide is 7-20 amino acids in length.

In certain embodiments, the presenting peptide is 8-10 amino acids in length.

In certain embodiments, the presenting peptide comprises an amino acid sequence as shown in SEQ ID NO: 17.

In certain embodiments, the presenting peptide comprises an amino acid sequence as shown in any one of SEQ ID NOs: 18-70.

In certain embodiments, the presenting peptide comprises an amino acid sequence as shown in SEQ ID NO: 35, SEQ ID NO: 30 or SEQ ID NO: 33.

In certain embodiments, the polynucleotide further comprises a nucleic acid molecule encoding a linker.

In certain embodiments, the presenting peptide and the B2M protein are linked via the linker in an expression product of the modified cell.

In certain embodiments, the linker comprises a rigid linker and/or a flexible linker.

In certain embodiments, the linker comprises an amino acid sequence as shown below: (EAAAK)n, where n is 3, 4, or 5.

In certain embodiments, the linker comprises an amino acid sequence as shown below: (GGGGS)n, where n is 3, 4, or 5.

In certain embodiments, the linker comprises an amino acid sequence as shown in any one of SEQ ID NOs: 71-99.

In certain embodiments, the linker comprises an amino acid sequence as shown in SEQ ID NO: 99.

In certain embodiments, the linker is located at the C-end of the presenting peptide.

In certain embodiments, the polynucleotide does not comprise a nucleic acid molecule encoding a B2M protein or a fragment thereof.

In certain embodiments, the polynucleotide encodes an amino acid sequence as shown in SEQ ID NO: 9.

In certain embodiments, the polynucleotide comprises a nucleotide sequence as shown in SEQ ID NO: 12.

In certain embodiments, the inserting is performed by introducing a vector comprising the polynucleotide.

In certain embodiments, the inserting comprises contacting a vector comprising the polynucleotide with the cell.

In certain embodiments, the vector comprising the polynucleotide does not comprise a complete B2M gene coding region.

In certain embodiments, the vector comprising the polynucleotide does not comprise a nucleic acid molecule encoding the heavy chain of the MHC molecule.

In certain embodiments, the vector comprising the polynucleotide further comprises a homology arm.

In certain embodiments, the vector comprising the polynucleotide comprises an upstream homology arm, the polynucleotide encoding the presenting peptide and a downstream homology arm.

In certain embodiments, the vector comprising the polynucleotide comprises a nucleotide sequence as shown in SEQ ID NO: 4.

In certain embodiments, the method further comprises introducing at least one sequence-specific agent that specifically targets the locus of the endogenous B2M gene into the cell.

In certain embodiments, the sequence-specific agent comprises a nuclease.

In certain embodiments, the sequence-specific agent comprises a RNA and/or DNA-guided endonuclease.

In certain embodiments, the endonuclease comprises a Cas protein.

In certain embodiments, the Cas protein comprises a Cas9 protein.

In certain embodiments, the sequence-specific agent further comprises a guide RNA (gRNA).

In certain embodiments, the gRNA is a single-stranded guide RNA.

In certain embodiments, the gRNA comprises a nucleotide sequence as shown in SEQ ID NO: 13.

In certain embodiments, the gRNA comprises 5'-(X)n-SEQ ID NO: 13-backbone sequence-3', where X is a base selected from any one of A, U, C and G, and n is any one integer of 0-15.

In certain embodiments, the gRNA comprises a nucleotide sequence as shown in SEQ ID NO: 3.

In certain embodiments, the modified cell is derived from a mammalian cell.

In certain embodiments, the modified cell is derived from a stem cell and/or a somatic cell.

In certain embodiments, the modified cell is derived from a blood cell, a muscle cell, a nerve cell, and/or an immune cell.

In certain embodiments, the modified cell is derived from an immune cell selected from the group comprising a natural killer (NK) cell, a T lymphocyte, a B lymphocyte, a NKT cell, a monocyte, a macrophage, a mast cell, a granulocyte, a dendritic cell, a lymphocyte, a leukocyte and/or a peripheral blood mononuclear cell.

In certain embodiments, the modified cell is a natural killer (NK) cell, a NKT cell and/or a T lymphocyte.

In certain embodiments, the expression of a classical MHC class I molecule on the cell surface of the modified cell is down-regulated, and the expression of a non-classical MHC class I molecule is positive.

In certain embodiments, the expression of a classical MHC class I molecule on the cell surface of the modified cell is down-regulated, and the expression of a non-classical MHC class I molecule is substantially not down-regulated.

In certain embodiments, the expression of a classical MHC class I molecule on the cell surface of the modified cell is down-regulated, and the expression of a non-classical MHC class I molecule is up-regulated.

In certain embodiments, the method further comprises down-regulating the expression and/or activity of a T cell receptor α constant region protein (TRAC), a T cell receptor β constant region protein (TRBC), CD3ε, CD3y, CD3δ and/or CD3ζ.

In certain embodiments, the method further comprises administering a guide RNA targeting a nucleic acid molecule encoding the TRAC to the cell.

In certain embodiments, the guide RNA targeting the nucleic acid molecule encoding the TRAC comprises an amino acid sequence as shown in SEQ ID NO: 8.

In certain embodiments, the method further comprises allowing the modified cell to express a chimeric antigen receptor (CAR), a T cell receptor (TCR), a chimeric autoantibody receptor (CAAR), a TCR receptor fusion construct (TRuC), a T cell antigen coupler (TAC), an antibody TCR receptor (AbTCR), a chimeric CD3ε receptor and/or at least one other synthetic receptor.

In certain embodiments, the method further comprises introducing a nucleic acid molecule encoding a chimeric antigen receptor (CAR), a T cell receptor (TCR), a chimeric autoantibody receptor (CAAR), a TCR receptor fusion construct (TRuC), a T cell antigen coupler (TAC), an antibody TCR receptor (AbTCR), a chimeric CD3ε receptor and/or at least one synthetic receptor into the modified cell.

In another aspect, the present application provides a modified cell prepared according to the method.

In another aspect, the present application provides an isolated nucleic acid molecule comprising the polynucleotide in the method.

In another aspect, the present application provides a guide RNA in the method.

In another aspect, the present application provides a nucleic acid combination comprising the isolated nucleic acid molecule and the guide RNA in the method.

In another aspect, the present application provides a vector comprising the isolated nucleic acid molecule and/or the nucleic acid combination.

In certain embodiments, the vector is a viral vector.

In certain embodiments, the vector is a lentiviral vector, an adenoviral vector, or an adeno-associated viral vector.

In another aspect, the present application provides a host cell comprising the isolated nucleic acid molecule, the guide RNA, the nucleic acid combination and/or the vector.

In another aspect, the present application provides a cell population comprising at least 30% of the modified cell.

In another aspect, the present application provides a pharmaceutical composition comprising the isolated nucleic acid molecule, the guide RNA, the nucleic acid combination, the vector, the modified cell, the host cell and/or the cell population, and optionally a pharmaceutically acceptable carrier.

In another aspect, the present application provides the use of the isolated nucleic acid molecule, the guide RNA, the nucleic acid combination, the vector, the modified cell, the cell, the cell population and/or the pharmaceutical composition in the preparation of a medicine for allogeneic transplantation.

In another aspect, the present application provides a method for allogeneic transplantation, the method comprises administering the isolated nucleic acid molecule, the guide RNA, the nucleic acid combination, the vector, the modified cell, the cell, the cell population and/or the pharmaceutical composition to a subject.

In another aspect, the present application provides a method for increasing allogeneic transplantation compatibility, the method comprises using the method described herein.

In another aspect, the present application provides the use of the isolated nucleic acid molecule, the guide RNA, the nucleic acid combination, the vector, the modified cell, the cell, the cell population and/or the pharmaceutical composition in the preparation of a medicine for the treatment or alleviation of a tumor.

In another aspect, the present application provides a method for treating or alleviating a tumor, the method comprises administering the isolated nucleic acid molecule, the guide RNA, the nucleic acid combination, the vector, the modified cell, the cell, the cell population and/or the pharmaceutical composition to a subject.

### Advantages of the present invention:

1. In terms of cell preparation, the knock-in expression efficiency of up to 70%-80% can be achieved based on the CRISPR Knock in method of the present invention. However, the HLA-E complex or CM co-expressed through CAR in the existing technology limits the transfection efficiency to between less than 20% and 50% due to large load capacity. Higher delivery efficiency makes CAR-T cells more efficient.
2. In terms of resisting immune rejection, unlike the prior art, the present invention does not knock out B2M and completely inactivates the HLA-ABC/B2M complex. When HLA-ABC/B2M expression is lost, although HLA-ABCB2M-negative cells can avoid killing by CD8-positive CTL, they are very sensitive to NK cells, and NK cells can quickly eliminate these HLA-ABC/B2M-negative cells. The present invention retains the B2M function. The state of high expression of HLA-E and at the same time weak expression of HLA-ABC is at a good balance point and can resist the killing by CTL and NK simultaneously, thereby achieving longer survival and proliferation of cells.

A person skilled in the art would have been able to easily notice other aspects and advantages of the present application from the detailed description below. Only exemplary embodiments of the present application are shown and described in the detailed description below. As a person skilled in the art will recognize, the content of the present application enables a person skilled in the art to make alterations to the disclosed specific embodiments without departing from the spirit and scope of the invention disclosed in the present application. Accordingly, the description in the drawings and description of the present application is merely exemplary rather than limiting.

### Brief Description of the Drawings

The specific features of the invention disclosed in the present application are as shown in the appended claims. The characteristics and advantages of the invention disclosed in the present application can be better understood with reference to the exemplary embodiments and drawings detailed herein below. A brief description of the drawings is as follows:
Fig. 1 shows a schematic diagram of the design of CRISPR/Cas9-mediated targeted insertion of HLA-E restricted presenting peptide into the B2M locus.
Fig. 2 shows the efficiency of targeted insertion and expression of HLA-E in primary T cells detected by flow cytometry.
Fig. 3 shows the detection of targeted insertion at genome-level by DNA sequencing, where A: HLA-ABC-HLA-E+ cell subset detected by flow cytometry (indicated by the arrow), B: DNA sequencing indicates targeted insertion of sequences.
Fig. 4 shows analysis of killing of target cells by NK92 by flow cytometry, where, A: flow cytometry analysis process, B: comparison of the changes in the proportion of HLA-E+ cell subset before and after killing by NK92.
Fig. 5 shows the evaluation of resistance to killing by NK92 in cells expressing HLA-E based on targeted insertion.
Fig. 6A shows the cell preparation process based on AAV delivery of homologous recombination templates.
Fig. 6B shows the expression of HLA-ABC and HLA-E in cells based on AAV delivery of homologous recombination templates.
Fig. 7 shows the UCAR-T cell preparation process based on AAV delivery of homologous recombination templates, where, A: preparation process of lentiviral delivery of CAR and AAV delivery of homologous recombination templates for site-specific insertion of HLA-E restricted presenting peptide in B2M gene; B: preparation process of AAV deliver of homologous recombination templates for site-specific insertion of CAR into the TRAC gene and site-specific insertion of HLA-E restricted presenting peptide into the B2M gene.
Fig. 8 shows the UCAR-T cell preparation process based on DNA delivery of homologous recombination templates, where, A: preparation process of lentiviral delivery of CAR and DNA delivery of homologous recombination templates for site-specific insertion of HLA-E restricted presenting peptide in B2M gene; B: preparation process of DNA deliver of homologous recombination templates for site-specific insertion of CAR into the TRAC gene and site-specific insertion of HLA-E restricted presenting peptide into the B2M gene.
Fig. 9 shows the comparison of double gene editing efficiency and CAR/HLA-E expression efficiency in UCAR-T cells prepared by different methods in Example 9 of the present application.
Fig. 10 shows the proliferation of CAR-positive cells in the allogeneic PBMC co-culture system of UCAR-T cells prepared by different methods in Example 9 of the present application.
Fig. 11 shows the detection results of the killing activity on tumor cells by UCAR-T cells prepared by different methods in Example 9 of the present application.

### Detailed Description of Embodiments

The embodiments of the present invention are illustrated below by using the specific examples, and those skilled in the art would have readily understood other advantages and effects of the present invention from the content disclosed in this description.

### Definition of Terms

In the present application, the term "modification" generally means that the state or structure of a molecule or cell is altered. Molecules can be modified in a variety of ways, including chemical, structural, and functional modifications. Cells can be modified by introducing polynucleotides. In the present application, the modification may be gene editing, and the modification alters the expression and/or activity of a protein in a cell.

In the present application, the term "presenting peptide" generally refers to a polypeptide capable of binding to a major histocompatibility complex (MHC) to form a complex. Presenting peptide can stabilize the stability and/or structural specificity of the MHC complex. After binding to the MHC, the presenting peptide can be presented on the cell surface. Each MHC molecule has its own matching presenting peptide. For different MHC molecules, the presenting peptide can be restricted, that is, a restricted presenting peptide for a specific MHC molecule means that the presenting peptide can form a stable complex with the specific MHC molecule. For example, a HLA-C-restricted presenting peptide can form a stable complex with HLA-C on the cell surface, and a HLA-E-restricted presenting peptide can form a stable complex with HLA-E on the cell surface. A restricted presenting peptide for a specific HLA can be identified by selecting a presenting peptide that binds stably to the HLA.

In the present application, the term "polynucleotide" generally refers to a single- or double-stranded polymer of deoxynucleotide or ribonucleotide bases. A single-stranded polynucleotide comprises a coding strand and an antisense strand. Polynucleotides include RNA and DNA, and can be isolated from natural sources, synthesized *in vitro,* or prepared from a combination of natural and synthetic molecules. Examples of polynucleotides include, but are not limited to, genes, cDNA, mRNA, self-replicating RNA molecules, self-replicating DNA molecules, genomic DNA sequences, genomic RNA sequences, or oligonucleotides. A polynucleotide can be linear or non-linear.

In the present application, the term "endogenous" when used to describe a molecule generally refers to a molecule that is naturally present in a cell or a product produced by a naturally occurring molecule in a cell through a physiological process that is naturally present in the cell (e.g., transcription or translation). In the present application, an "endogenous" molecule may be a molecule present in a cell without the modification (*i.e.,* before the modification) or a natural product thereof. With respect to a nucleic acid, "endogenous" may refer to the genome sequence of a cell that is naturally present at a locus or present in a natural content.

The opposite of "endogenous" is "exogenous". An "exogenous" molecule usually refers to a molecule that is not naturally present in a cell. An exogenous molecule can be introduced into a cell by one or more methods, such as genetic methods, biochemical methods, or other methods. An exogenous molecule: (a) may be foreign to a given cell (*i.e.,* exogenous to the cell); (b) may be naturally present in a cell (*i.e.,* "endogenous"), but is present in the cell in an unnatural amount (*i.e*., greater or less than the amount naturally present in the cell); or (c) may be naturally present in a host cell but outside its natural locus. Exogenous molecules may be small molecules produced by, for example, combinatorial chemistry, or macromolecules (such as proteins, nucleic acids, carbohydrates, lipids, glycoproteins, lipoproteins, polysaccharides, any modified derivatives of the above molecules), or any complex comprising one or more of the above molecules.

An exogenous molecule may be the same type of molecule as a endogenous molecule, for example, a protein or nucleic acid (including DNA and/or RNA). For example, an exogenous nucleic acid may comprise an infectious viral genome, a plasmid or episomal gene introduced into a cell, or a chromosome not normally present in a cell. Methods for introducing exogenous molecules into cells are known to those skilled in the art and include, but are not limited to, lipid-mediated transfer (*i.e.,* liposomes, including neutral lipids and cationic lipids), electroporation, direct Injection, cell fusion, particle bombardment, biopolymer nanoparticle delivery (see Nitta and Numata (2013) Int J Mol Sci 14:1629), calcium phosphate coprecipitation, DEAE-polydextrose mediated transfer, and viral vector mediated transfer. An exogenous molecule may also be a molecule of the same type as an endogenous molecule but derived from a cell of a different origin.

With respect to a nucleic acid, "exogenous" generally refers to any nucleotide or nucleic acid sequence that is not present naturally at a particular locus. For example, the polynucleotide inserted by the method of the present application is referred to as an "exogenous" polynucleotide. Insertion of an "exogenous" polynucleotide may produce a gene editing effect on an endogenous sequence. The nucleic acid sequences of an "exogenous" polynucleotide and an "endogenous" polynucleotide may be partially or completely identical, or may encode partially or completely identical amino acid sequences.

In the present application, the term "locus" generally refers to a specific location of a DNA sequence (for example, a gene) in the genome. A locus may comprise a genetic element encoding information for a protein and/or a regulatory element involved in the transcription and translation of a gene.

In the present application, the term "endogenous B2M locus" generally refers to a B2M locus originally present in the genome of a cell without the modification as described herein. For example, an endogenous B2M locus can correspond to the position Chr15:44,711,487-44,718,851 and its vicinity in the GRCh38/hg38 version of the human reference genome (see the Ensembl database ENSG00000166710), including a B2M promoter, a transcriptional regulatory factor and other positions, such as Fishilevich , S. et al. GeneHancer: genome-wide integration of enhancers and target genes in GeneCards. Database (Oxford). 2017, 10.1093/database/bax028 (2017). B2M promoter and enhancer locations displayed in the database are: such as chr15:44705644-44717291 (GRCh38/hg38), chr15:44661600-44667653 (GRCh38/hg38), chr15:44723092-44732751 (GRCh38/hg38), chr15:45047617-45050241 (GRCh38/hg38), chr15:44783825-44785452 (GRCh38/hg38) *etc.* For example, the endogenous B2M locus can also be referred to the description under NCBI Gene ID: 567, and the location of the endogenous B2M locus can correspond to GRCh38.p13 (GCF_000001405.39), chromosome 15, NC_000015.10 (44711492..44718145), and its vicinity. Persons in the art should understand that when different classification methods or rules are used, there may be one or more base differences in locus positions.

In the present application, the term "transcriptional regulation" generally means that a regulatory element can induce, repress, or regulate the transcription of a protein-coding sequence to which it is operably linked. Regulatory elements capable of transcriptional regulation can include initiation sequences, enhancers, promoters, terminators, efficient RNA processing signals (such as splicing and polyadenylation signals), sequences that stabilize cytoplasmic mRNA, sequences that improve translation efficiency (*i.e.,* Kozak consensus sequence), sequences that increase protein stability and, if necessary, sequences that increase protein secretion.

In the present application, when the term" under the transcriptional regulation of the endogenous B2M locus" is used to modify a certain polynucleotide, it usually means that the transcription of the polynucleotide is regulated by a regulatory element in the endogenous B2M locus.

In the present application, the term "B2M" may also be referred to as "β-2 microglobulin" and generally refers to a light chain of a MHC class I molecule. The "B2M" includes any natural B2M from any vertebrate source, including mammals, such as primates (for example, humans and cynomolgus monkeys) and rodents (for example, mice and rats). The term "B2M" encompasses "full length" B2M, unprocessed B2M as well as any form of B2M resulting from cellular processing. "B2M" includes complete B2M1 and fragments thereof, and also includes functional variants, isoforms, species homologs, derivatives and analogs of B2M. B2M (also known as β chain) can combine with HLA-encoded α chain (or heavy chain) to form a MHC class I molecule. In the human genome, B2M proteins are generally encoded by the b2m gene located on chromosome 15. The human full-length B2M protein usually has 119 amino acids, of which amino acids 1 to 20 are signal peptides (see UniProt database code P61769). Exemplary functional fragments of β2M include, for example, ΔN6β2M (93 amino acids), a truncated form that lacks the 6 N-terminal amino acids of mature β2M, and ΔN10β2M that lacks the 10 N-terminal amino acids (89 amino acids). Exemplary variants of β2M may include, for example, the following isoforms available on UniProt: Entry F5H6I0 (101 amino acids), H0YLF3 (71 amino acids), B4E0X1 (122 amino acids), A6XMH4 (124 amino acids), A6XMH5 (92 amino acids), A6XND9 (101 amino acids), Q9UM88 (29 amino acids), Q16446 (51 amino acids) and J3KNU0 (57 amino acids).

In the present application, the term "B2M mature protein" generally refers to one or more forms of B2M that do not have a 20-amino acid signal peptide that can be cleaved before B2M is expressed on the cell surface. The amino acid sequence of an exemplary B2M mature protein may be the amino acid sequence as shown in SEQ ID NO: 15.

In the present application, the term "B2M protein fragment" generally refers to a portion of the full-length B2M protein amino acid sequence. For the full-length human B2M protein with a length of 119 amino acids, the B2M protein fragment comprises any length from 118 amino acids to 1 amino acid, but does not comprises all the 119 amino acids.

In the present application, the term "down-regulation" generally refers to the reduction or elimination of the level and/or activity of nucleic acid molecules (for example, RNA or DNA) encoding one or more protein or functional fragments thereof, or the level and/or activity of one or more protein or functional fragments thereof in immune cells modified by the present application, to a level and/or activity which is lower than that in immune cells not modified by the present application.

In the present application, the term "substantially not down-regulated" generally means that the level of expression and/or activity of B2M protein on the cell surface of experimental cells (for example, modified cells) is equivalent or does not produce significant down-regulation compared with reference cells (for example, cells without the modification). For example, "substantially not down-regulated" may mean that the expression and/or activity of B2M protein on the cell surface of experimental cells (for example, modified cells) is down-regulated by no more than about 80%, about 70%, about 60%, about 50%, about 40%, about 30%, about 20% or less compared with reference cells (for example, cells without the modification).

In the present application, the term "covalently bind" generally refers to the covalent bonding between two polypeptides via amino acids.

In the present application, the term "complete B2M gene coding region" generally refers to the gene encoding the entire B2M mature protein. For example, the "complete B2M gene coding region" encodes the mature B2M protein of 99 amino acids in length.

In the present application, the term "heavy chain of MHC molecule" generally refers to the longer chain of a MHC class I molecule, which may also be called the α chain. The heavy chain of an MHC molecule may include a peptide-binding region, an immunoglobulin-like region, a transmembrane region, and an intracellular region.

In the present application, the term "open reading frame" generally refers to a nucleic acid sequence encoding a protein without interrupted by a terminating sequence. The open reading frame starts from a start codon and ends with a stop codon. The open reading frame may also be called reading frame.

In the present application, the term "operably linked" generally refers to placing a regulatory sequence necessary for the expression of the coding sequence into an appropriate position relative to the coding sequence in order to achieve expression of the coding sequence. For example, a first nucleic acid sequence is operably linked to a second nucleic acid sequence when the first nucleic acid sequence is in a functional relationship with the second nucleic acid sequence. For instance, a promoter is operably linked to a coding sequence if the promoter affects the transcription or expression of the coding sequence. Typically, operably linked DNA sequences are contiguous and two protein coding regions must be joined in the same open reading frame.

In the present application, the term "allogeneic rejection response" generally refers to immunological responses in which the cells, tissues or organs from one individual are recognized by the immune system of another individual, and the immune system attacks, destroys and eliminates the cells, tissues or organs from different individuals. Different individuals can be different individuals of the same species, or different individuals of different species.

In the present application, the term "MHC complex" is also commonly referred to as major histocompatibility complex (MHC), which is a collective name for a group of genes encoding major histocompatibility antigens in animals. Human MHC is also called HLA (human leukocyte antigen, HLA) complex. Due to the polygenic characteristics of MHC, MHC can be divided into MHC class I, MHC class II and MHC class III genes according to the structure, tissue distribution and functional differences of their coding molecules, which gens encode MHC class I molecules, MHC class II molecules and MHC class III molecules respectively. MHC class I molecules usually consist of four domains, three of which are located on the α chain (α1-α3), and β2 microglobulin (B2M) constitutes the fourth domain.

In the present application, the term "immune cell activation" generally refers to the state in which an immune response is generated for a given type of immune cell. For example, for T cells, characteristics of T cell activation typically include one or more of the following changes: producing a variety of proteins (including CD69, CD7, and CD25 and HLA-DR), releasing perforin, granzyme and granulysin (degranulation) or producing cytokines (such as IFN-y, TNF and LT-α). For example, for T cells, characteristics of T cell activation typically include one or more of the following changes: releasing perforin, granzyme and granulysin (degranulation), and producing cytokines (such as IFN-y, TNF, GM-CSF, IL-3, and M-CSF).

In the present application, the term "inhibitory receptor" generally refers to a receptor molecule expressed on the surface of immune cells that can inhibit immune cell activation upon binding to its ligand. The immune system employs multiple inhibitory mechanisms to control immune responses to ensure immune tolerance and homeostasis, including through inhibitory receptor-mediated inhibitory pathways. For example, NK cells are regulated by autologous MHC class I -binding receptors, which inhibit NK cell activation upon binding to autologous MHC class I molecules. MHC class I-binding inhibitory receptors can be expressed not only on NK cells but also on T cell subsets, for example, CD8⁺ T cells. In the present application, the inhibitory receptors generally refer to receptors specific for MHC class I molecules. These inhibitory receptors may include the killer immunoglobulin-like receptor (KIR) family and the C-type lectin-like family. For example, the inhibitory receptors may include NKG2A/KLRD1 (CD159a/CD94), KIR2DL1 (CD158a), KIR2DL2 (CD158b), KIR2DL3 (CD158d), KIR2DL5 (CD158f), KIR3DL1 (CD158e1), KIR3DL2 (CD158k), ILT2/LIR-1 (CD85J), LAG-3 (CD223) and/or KIR2DL4 (CD158d).

In the present application, the term "non-classical MHC class I molecule" generally includes HLA-E, HLA-F, HLA-G, *etc.* HLA-E molecules have multiple alleles and can be expressed in various tissue cells and are highly expressed on the surface of amniotic membrane and trophoblast cells. HLA-E molecules are specific ligands of the CD94/NKG2 family in the C-type lectin receptor superfamily expressed on the surface of NK cells and some CTLs. HLA-G molecules are mainly distributed in extravillous trophoblast cells at the maternal-fetal interface, and the corresponding receptors are members of the killer immunoglobulin-like receptor (KIR) family, so HLA-G can play a role in maternal-fetal tolerance. Like classical MHC class I molecules, nonclassical MHC class I molecules can include heavy chains and light chains, in which the heavy chains of nonclassical MHC class I molecules can include HLA-E heavy chains, HLA-F heavy chains and HLA-G heavy chains.

In the present application, the term "MHC-like molecule" generally refers to molecules that have a similar conformation to MHC class I molecules but bind to different types of ligands and cell surface receptors. Like MHC class I molecules, MHC-like molecules can include heavy chains and light chains, in which the heavy chains of MHC-like molecules can include human leukocyte antigen (HLA-1), neonatal Fc receptor (FcRn), hereditary hemochromatosis protein (HFE), cluster of differentiation 1 (CD1), γ δ T cell receptor ligand (T22), zinc-α2-glycoprotein (ZAG), MHC-related protein 1 (MR1), UL18, UL16-binding protein (ULBP), and MH.

In the present application, the term "immune cell" generally refers to cells that are functionally involved in the initiation and/or progression of innate and/or adaptive immune responses. The immune cells may be of hematopoietic origin. The immune cells may be dendritic cells, killer dendritic cells, mast cells, NK cells, B cells, or T cells selected from inflammatory T lymphocytes, cytotoxic T lymphocytes, regulatory T lymphocytes or helper T lymphocytes. Cells can be obtained from many non-limiting sources, including peripheral blood mononuclear cells, bone marrow, lymph node tissue, umbilical cord blood, thymus tissue, tissues from sites of infection, ascites, pleural effusion and spleen tissues, and from tumors, such as tumor-infiltrating lymphocytes. For example, the immune cells can be from a healthy donor, from a patient diagnosed with a cancer, or from a patient diagnosed with an infection.

In the present application, the term "linker" generally refers to a moiety that joins or connects two or more discrete and separate monomeric domains. Linkers of the present application may be peptide linkers, including those of natural and/or synthetic origin. The linker may consist of a linear chain of amino acids, of which the 20 naturally occurring amino acids are the monomeric building blocks. The linker may be 1-50 amino acids in length, for example, may be between 1 and 28 amino acids in length, for example, may be between 3 and 25 amino acids in length. The linker may comprise a repeating amino acid sequence, or a naturally occurring polypeptide sequence, such as a polypeptide that functions as a hinge. By allowing the correct folding and proper presentation of the peptide, the linker, which functions to link the B2M protein to the heavy chain of the MHC molecule, can carry out its biological activity. For example, linkers can be designed to be rich in glycine, glutamic acid, and/or serine residues. These residues are arranged, for example, into small a repeating unit of up to 5 amino acids, such as GGGGS (SEQ ID NO: 71), QQQQG (SEQ ID NO: 72) or SSSSG (SEQ ID NO: 73). This small repeating unit can be repeated 2-5 times to form a multimeric unit. At the amino- and/or carboxyl terminus of the multimeric unit, up to 6 additional optional, naturally occurring amino acids can be added. Other synthetic peptide linkers include a single amino acid repeated between 10 times and 20 times and may include up to 6 additional optional, naturally occurring amino acids at the amino- and/or carboxyl-terminus. For example, the linker may include, but not limited to, the amino acid sequence as shown in any one of SEQ ID NOs: 71-99.

In the present application, the term "specific targeting" generally refers to the ability to recognize a certain nucleic acid sequence but not a sequence that is not the nucleic acid sequence, or to recognize a certain nucleic acid sequence more than a sequence that is not the nucleic acid sequence.

In the present application, the term "sequence-specific agent" generally refers to any active molecule that has the ability to specifically recognize a selected target sequence at a genomic locus. For example, the sequence-specific agent may be a sequence-specific nuclease agent. Nucleic acid sequences that can be recognized by the sequence-specific agent are called "target sequences".

In the present application, the term "nuclease agent" generally refers to a nucleic acid molecule that contributes to a nuclease-catalyzed reaction (e.g., endonuclease reaction) in a target cell by itself or as a subunit of a complex (e.g., guide RNA/Cas9).

In the present application, the term "Cas protein", also known as "CRISPR-related protein", generally refers to a class of enzymes that are complementary to the CRISPR sequence and is able to use the CRISPR sequence as a guide to recognize and cleave specific DNA strands. Non-limiting examples of Cas proteins include: Casl, CaslB, Cas2, Cas3, Cas4, Cas5, Cas6, Cas7, Cas8, Cas9 (also known as Csnl and Csxl2), CaslO, Csyl, Csy2, Csy3, Csel, Cse2, Cscl, Csc2, Csa5, Csn2, Csm2, Csm3, Csm4, Csm5, Csm6, Cmrl, Cmr3, Cmr4, Cmr5, Cmr6, Csbl, Csb2, Csb3, Csxl7, Csxl4, CsxlO, Csxl6, CsaX, Csx3, Csxl, Csxl5, Csf1, Csf2, Csf3, Csf4, and/or their homologues, or modified forms thereof. In some embodiments, the Cas protein is a Cas9 protein.

In the present application, the term "Cas9 protein" or "Cas9 nuclease", also known as Csn1 or Csx12, generally refers to a class of proteins in type II CRISPR/Cas systems that are involved in both crRNA biosynthesis and destruction of invading DNA. Cas9 proteins usually include RuvC nuclease domain and HNH nuclease domain, which cleave two different strands of double-stranded DNA molecules respectively. Cas9 proteins can be derived from *S. thermophiles, Listeria innocua,* and *Streptococcus pyogenes.* For example, *Streptococcus pyogenes* Cas9 protein, its amino acid sequence can be found in the SwissProt database accession number Q99ZW2; *Neisseria meningitides* Cas9 protein, its amino acid sequence can be found in UniProt database number A1IQ68; *Streptococcus thermophilus* Cas9 protein, its amino acid sequence can be found in the UniProt database number Q03LF7; and *Staphylococcus aureus* Cas9 protein, its amino acid sequence can be found in the UniProt database number J7RUA5.

In the present application, the term "guide RNA" generally refers to the RNA component contained in CRISPR, which may also be called gRNA. Guide RNA generally comprises a guide sequence and a backbone sequence, wherein the guide sequence and the backbone sequence may be in the same molecule or in different molecules. The role of the guide RNA can be to direct the Cas9 protein to cleave the DNA site complementary to the guide sequence, *i.e.,* the target sequence. In general, a guide sequence is any polynucleotide sequence having sufficient complementarity with a target sequence to hybridize with this target sequence and direct the specific binding of a CRISPR complex to the target sequence. The degree of complementarity between a guide sequence and its corresponding target sequence is about or more than about 50%, or more. In general, a guide sequence is about or more than about 12 nucleotides in length. The backbone sequence is necessary for guide RNA. The sequences other than the guide sequence generally comprise a tracr sequence and a tracr mate sequence, which generally do not vary with changes in the target sequence. The term includes single-stranded guide RNA (sgRNA) as well as double-stranded guide RNA composed of crRNA (CRISPR RNA) and tracrRNA (trans-activating crRNA).

In the present application, the term "signal peptide" generally refers to a peptide present at the N-terminus of a protein precursor. The signal peptide may function to facilitate translocation of the expressed polypeptide linked to the endoplasmic reticulum. The signal peptide is usually cleaved during protein expression to the surface. A signal peptide may be heterologous or homologous to the organism used to produce the polypeptide. The amino acid sequence of the signal peptide of the B2M protein can be shown as SEQ ID NO: 16.

In the present application, the term "synthetic receptor" generally refers to a cell surface protein or protein complex that may comprise (1) a target binding domain that can specifically bind a target molecule, and (2) a functional domain that can activate a signaling pathway. In the synthetic receptor, the target binding domain may comprise an extracellular domain and the functional domain that activates the signaling pathway may comprise an intracellular domain. The synthetic receptor may also include a transmembrane sequences. The synthetic receptor can be a protein complex comprising a protein expressed from an exogenous nucleic acid. The synthetic receptor may also be a protein complex comprising at least one exogenously expressed protein and at least one endogenously expressed protein. In certain embodiments, the synthetic receptor can be selected from: a chimeric antigen receptor (CAR), a T cell receptor (TCR), a chimeric autoantibody receptor (CAAR), a TCR receptor fusion construct (TRuC), a T cell antigen coupler (TAC), an antibody TCR receptor (AbTCR) and a chimeric CD3ε receptor. In some embodiments, the synthetic receptor may be a CAR. In some embodiments, the synthetic receptor may be a TCR. In some embodiments, the synthetic receptor may be a CAAR. In some embodiments, the synthetic receptor may be TRuC. In some embodiments, the synthetic receptor may be a TAC. In some embodiments, the synthetic receptor may be AbTCR. In some embodiments, the synthetic receptor may be a chimeric CD3ε receptor.

In the present application, the term "isolated" generally refers to a biological material (for example, a virus, a nucleic acid, or a protein) that is substantially free of components that normally accompany or interact with it in its naturally occurring environment. The isolated biological material optionally comprises an additional material that the biological material is not found to possess in its natural environment (for example, a cell or a wild-type virus). For example, if the material is in its natural environment (for example, a cell), the material may have been placed in a location in the cell (for example, genome or genetic component) that is not native to the material in that environment. For example, if a naturally occurring nucleic acid (for example, a coding sequence, a promoter, and an enhancer) is introduced into a locus of a genome that is not native to that nucleic acid (for example, a vector (for example, a plasmid or viral vector) or an amplicon) by a non-naturally occurring means, then the naturally occurring nucleic acid is isolated. Such a nucleic acid is also referred to as a "heterologous" nucleic acid. The isolated virus is in an environment (for example, a cell culture system or purified from cell culture) that is different from the natural environment of the wild-type virus (for example, the nasopharynx of an infected individual).

In the present application, the "vector" generally refers to a nucleic acid molecule capable of self-replication in a suitable host for the purpose of transferring the inserted nucleic acid molecule into and/or between host cells. The vector may comprise a vector primarily used for inserting DNA or RNA into a cell, a vector primarily used for replicating DNA or RNA, and a vector primarily used for the transcriptional and/or translational expression of DNA or RNA. The vector also comprises a vector having a variety of the above-mentioned functions. The vector can be a polynucleotide that can be transcribed and translated into a polypeptide when introduced into a suitable host cell. Generally, the vector may produce a desired expression product by means of culturing a host suitable cell containing the vector.

In the present application, the term "plasmid" generally refers to a DNA molecule other than the chromosome or nucleoid in bacteria, yeast and other organisms, which exists in the cytoplasm and has the ability to replicate autonomously, enabling it to maintain a constant copy number in progeny cells and express the genetic information it carries. Plasmids are used as carriers of genes in genetic engineering research.

In the present application, the term "pharmaceutical composition" generally refers to a formulation in a form that is effective in allowing the biological activity of the active ingredient and does not contain additional ingredients that would be unacceptable toxicity to the subject to whom the formulation is to be administered.

In the present application, the term "cells without modification" generally refers to cells into which a polynucleotide encoding a presenting peptide described herein has not been inserted. Cells without the modification may undergo other chemical or biological modifications, such as up-regulation or down-regulation of one or more genes, expression of other exogenous molecules that are not the presenting peptide, or glycosylation modifications.

In the present application, the term "increasing allogeneic transplantation compatibility" generally refers to, during allogeneic transplantation, increasing the acceptance of the allograft by the recipient's immune system, or reducing responses of attacking or eliminating the allograft by the recipient's immune system.

In addition to the specific proteins and nucleotides mentioned herein, the present application may also comprise functional variants, derivatives, analogs, homologs, and fragments of the proteins and nucleotides.

The term "functional variant" refers to a polypeptide that has an amino acid sequence substantially identical to a naturally occurring sequence, or is encoded by a substantially identical nucleotide sequence and can have one or more activities of a naturally occurring sequence. In the context of the present application, a variant of any given sequence refers to a sequence in which a specific sequence of a residue (whether an amino acid residue or a nucleotide residue) has been modified so that the polypeptide or the polynucleotide substantially retains at least one endogenous function. A variant sequence can be obtained by means of the addition, deletion, substitution, modification, replacement and/or variation of at least one amino acid residue and/or nucleotide residue present in a naturally occurring protein and/or polynucleotide, as long as the original functional activity can be maintained.

In the present application, the term "derivative" generally refers to the polypeptide or polynucleotide of the present application, comprising any substitution, variation, modification, replacement, deletion, and/or addition of one or more amino acid residues from/on a sequence, as long as the obtained polypeptide or polynucleotide substantially retains at least one endogenous function thereof.

In the present application, for a polypeptide or polynucleotide, the term "analog" generally comprises any mimics of the polypeptide or polynucleotide, that is, a chemical compound that possesses at least one endogenous function of the polypeptide or polynucleotide simulated by the mimic.

Generally, amino acid substitution can be performed, for example, at least one (e.g., 1, 2, 3, 4, 5, 6, 7, 8, 9, 10, or more than 20) amino acid substitution, as long as a modified sequence substantially retains the desired activity or ability. Amino acid substitution may comprise the use of a non-naturally occurring analog.

Proteins or peptides used in the present application may also have the deletion, insertion or substitution of amino acid residues, and the amino acid residues produce silent changes and lead to functionally equivalent proteins. Intentional amino acid substitution can be performed on the basis of the similarity between the polarity, charge, solubility, hydrophobicity, hydrophilicity, and/or amphipathicity of residues, as long as the endogenous function is retained. For example, negatively charged amino acids comprise aspartic acid and glutamic acid; positively charged amino acids comprise lysine and arginine; and amino acids containing uncharged polar head groups with similar hydrophilic values comprise asparagine, glutamine, serine, threonine, and tyrosine.

In the present application, the term "homolog" generally refers to an amino acid sequence or a nucleotide sequence having a certain homology to the amino acid sequence of wild-type and the nucleotide sequence of wild-type. The term "homology" can be equivalent to sequence "identity". A homologous sequence may comprise an amino acid sequence that can be at least 80%, 85%, 90%, 99.1%, 99.2%, 99.3%, 99.4%, 99.5%, 99.6%, 99.7%, 99.8% or 99.9% identical to the subject sequence. Generally, homologs will contain active sites, etc. that are identical to the subject amino acid sequence. Homology can be considered on the basis of similarity (i.e., amino acid residues having similar chemical properties/functions), or can be represented in terms of sequence identity. In the present application, a sequence having a percentage identity to any one of the amino acid sequence or nucleotide sequence of the mentioned SEQ ID NO refers to a sequence having the percentage identity over the full-length sequence of the mentioned SEQ ID NO.

To determine a sequence identity, sequence alignment can be performed by using various methods known to a person skilled in the art, for example, using softwares such as BLAST, BLAST-2, ALIGN, NEEDLE or Megalign (DNASTAR). A person skilled in the art can determine appropriate parameters for alignment, including any algorithms required for achieving optimal alignment over the compared full-length sequences.

In the present application, the term "and/or" should be understood to mean either one of optional items or both of the optional items.

In the present application, the terms "contain", "contains" and "containing" generally refer to comprising explicitly specified features, but not excluding other elements.

In the present application, the term "approximately" generally refers to a change within a range of 0.5%-10% above or below a specified value, for example, a change within a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below a specified value.

### Detailed Description of the Invention

In one aspect, the present application provides a modified cell comprising a polynucleotide that encodes a presenting peptide, wherein the polynucleotide is under the transcriptional regulation of an endogenous B2M gene. In the present application, the expression of the polynucleotide can be transcriptionally regulated by the endogenous B2M gene. In the present application, the expression of the polynucleotide can be regulated by a regulatory element in the endogenous B2M locus. In the present application, the expression of the polynucleotide can be regulated by a promoter, an enhancer, a terminator, poly A, a signal peptide and/or a kozak sequence in the endogenous B2M locus. In the present application, the expression of the polynucleotide can be operably linked to a promoter, an enhancer, a terminator, poly A, a signal peptide and/or a kozak sequence in the endogenous B2M locus.

In the present application, the expression of the polynucleotide and the B2M gene may be regulated by the same promoter, for example, by the endogenous promoter of the B2M gene. In the present application, the expression of the polynucleotide and the B2M gene may be regulated by the same enhancer, for example, by the endogenous enhancer of the B2M gene. In the present application, the expression of the polynucleotide and the endogenous B2M gene may be regulated by the same poly A, for example, by the endogenous poly A of the B2M gene. In the present application, the expression of the polynucleotide and the endogenous B2M gene may be regulated by the same terminator, for example, by the endogenous terminator of the B2M gene. In the present application, the polynucleotide is located in the locus of the endogenous B2M gene and is regulated by the endogenous promoter of the B2M gene. In the present application, the polynucleotide can be operably linked to the endogenous promoter of the B2M gene. In the present application, the polynucleotide may be located downstream of the endogenous promoter of the B2M gene. In the present application, the polynucleotide may be located between a nucleic acid molecule encoding the endogenous signal peptide of the B2M gene and a nucleic acid molecule encoding the B2M mature protein.

### B2M

In the present application, the modified cell can express the B2M protein based on the special design of the polynucleotide insertion site and the insertion sequence. In the present application, the modified cell can express the B2M protein on the cell surface. In the present application, the modified cell can express the B2M protein on the cell surface, and at least a portion of the B2M protein can be expressed by the endogenous B2M gene. In the present application, the modified cell can express the B2M protein on the cell surface, and at least a portion (for example, at least 1%, 5%, 10%, 15%, 20%, 25%, 30 %, 35%, 40%, 45%, 50%, 55%, 60%, 65%, 70%, 75%, 80%, 85%, 90%, 95%, 98%, 99% or more) of the B2M protein can be expressed by the endogenous B2M gene. In the present application, the modified cell can express the B2M protein on the cell surface, and at least a portion (for example, at least 1%, 5%, 10%, 15%, 20%, 25 %, 30%, 35%, 40%, 45%, 50%, 55%, 60%, 65%, 70%, 75%, 80%, 85%, 90%, 95%, 98%, 99% or more) of the gene encoding the B2M protein can come from the endogenous B2M gene. When the polynucleotide contained in the cell comprises a fragment encoding the B2M protein, a portion of the expressed complete B2M protein is expressed by the polynucleotide, and the remaining portion is expressed by the endogenous B2M gene. In this case, a portion of the B2M gene in the cell can be deleted (a variety of known methods for gene deletion can be used, such as gene editing), and the deleted portion can be supplemented by the insertion of exogenous polynucleotides. At this time, the exogenous polynucleotide is inserted into the position of deletion of the B2M gene. At this time, the exogenous polynucleotide and the non-deleted endogenous B2M co-express the complete B2M protein.

In the present application, the modified cells can express the B2M protein on the cell surface, and the B2M protein can be substantially expressed by the endogenous B2M gene. For example, the complete B2M protein may be expressed from the endogenous B2M gene. For example, the genes encoding the B2M protein may all be derived from the endogenous B2M gene. At this time, the inserted exogenous polynucleotide does not comprise the complete B2M coding region. Alternatively, the inserted exogenous polynucleotide may not comprise a nucleic acid molecule encoding a full-length B2M protein.

In the present application, the expression and/or activity of the B2M protein on the cell surface may be substantially not down-regulated compared with cells without the modification. In the present application, the expression and/or activity of the B2M protein on the cell surface may be down-regulated by no more than about 80%, about 70%, about 60%, about 50%, about 40%, about 30%, about 20% or less compared with cells without the modification. In the present application, insertion of the polynucleotide into a cell does not substantially affect the expression of the B2M protein on the cell surface. In the present application, insertion of the polynucleotide into a cell allows the expression of the B2M protein on the cell surface to be down-regulated by no more than about 80%, about 70%, about 60%, about 50%, about 40%, about 30%, about 20% or less.

### heavy chain of MHC molecule

In the present application, the modified cell can express the presenting peptide, which can be covalently linked to the B2M protein to form a fusion protein, and the fusion protein can form a complex with the heavy chain of the MHC molecule on the cell surface. In the complex, the presenting peptide can bind to the peptide binding groove of the heavy chain of the MHC molecule.

By inserting different types of presenting peptides into cells, modified cells with different functions can be obtained. The type of the heavy chain of the MHC molecule can be selected according to the required function, thereby selecting the restricted presenting peptide of the MHC molecule. In the present application, the polynucleotide inserted into the cell may not comprise a nucleic acid molecule encoding the heavy chain of the MHC molecule. For example, some complexes can inhibit immune cell activation. In the present application, the complex can inhibit the immune cells from participating in the immune response by binding to inhibitory receptors on the surface of immune cells. The inhibiting immune cells from participating in the immune response may include inhibiting immune cell differentiation, proliferation or secretion of cytokines.

In the present application, the heavy chain of the MHC molecule may be a heavy chain of a non-classical MHC class I molecule and/or a heavy chain of an MHC-like molecule.

In the present application, the presenting peptide can form a fusion protein with the B2M protein, and the fusion protein can bind to a heavy chain of a non-classical MHC class I molecule to form a complex that binds to inhibitory receptors of immune cells. The heavy chain of the non-classical MHC class I molecule may be selected from a HLA-E heavy chain, a HLA-F heavy chain and a HLA-G heavy chain.

In the present application, the heavy chain of the non-classical MHC class I molecule may be an HLA-E heavy chain. In the present application, the presenting peptide can form a fusion protein with the B2M protein, and the fusion protein can bind to the HLA-E heavy chain to form a complex that binds to inhibitory receptors of immune cells. In the complex, the presenting peptide can bind to the peptide binding groove of the HLA-E heavy chain. In the present application, the polynucleotide inserted into the cell may not comprise a nucleic acid molecule encoding the HLA-E heavy chain. Human HLA-E heavy chains are polymorphic. For example, the amino acid sequence of one allelic form of the human HLA-E heavy chain can be found in Uniprot accession number Q6DU50.

In the present application, the heavy chain of the non-classical MHC class I molecule may be an HLA-F heavy chain. In the present application, the presenting peptide can form a fusion protein with the B2M protein, and the fusion protein can bind to the HLA-F heavy chain to form a complex that binds to inhibitory receptors of immune cells. In the complex, the presenting peptide can bind to the peptide binding groove of the HLA-F heavy chain. In the present application, the polynucleotide inserted into the cell may not comprise a nucleic acid molecule encoding the HLA-F heavy chain. Human HLA-F heavy chains are polymorphic. For example, the amino acid sequence of one allelic form of the human HLA-F heavy chain can be found in Uniprot accession number P30511.

In the present application, the heavy chain of the non-classical MHC class I molecule may be an HLA-G heavy chain. In the present application, the presenting peptide can form a fusion protein with the B2M protein, and the fusion protein can bind to the HLA-G heavy chain to form a complex that binds to inhibitory receptors of immune cells. In the complex, the presenting peptide can bind to the peptide binding groove of the HLA-G heavy chain. In the present application, the polynucleotide inserted into the cell may not comprise a nucleic acid molecule encoding the HLA-G heavy chain. Human HLA-G heavy chains are polymorphic. For example, the amino acid sequence of one allelic form of the human HLA-G heavy chain can be found in Uniprot accession number P17693.

In the present application, the presenting peptide can form a fusion protein with the B2M protein, and the fusion protein can bind to the heavy chain of an MHC-like molecule to form a complex that binds to inhibitory receptors of immune cells. The heavy chain of the MHC-like molecule may be selected from a HLA-E heavy chain, a HLA-F heavy chain and a HLA-G heavy chain may be selected from CD1, MR1, FcRN and UL18.

In the present application, the heavy chain of the MHC-like molecule may be a CD1 heavy chain. In the present application, the presenting peptide can form a fusion protein with the B2M protein, and the fusion protein can bind to the CD1 heavy chain to form a complex that binds to inhibitory receptors of immune cells. In the complex, the presenting peptide can bind to the peptide binding groove of the CD1 heavy chain. In the present application, the polynucleotide inserted into the cell may not comprise a nucleic acid molecule encoding the CD1 heavy chain. A human CD1 heavy chains may include a CD1a heavy chain, a CD1b heavy chain, a CD1c heavy chain, a CD1d heavy chain and a CD1e heavy chain. For example, the amino acid sequence of the human CD1a heavy chain can be found in NCBI accession number NP_001754.2. For example, the amino acid sequence of the human CD1b heavy chain can be found in NCBI accession number XP_011508421.1. For example, the amino acid sequence of the human CD1c heavy chain can be found in NCBI accession number XP_005245636.1. For example, the amino acid sequence of the human CDId heavy chain can be found in NCBI accession number NP_001358692.1. For example, the amino acid sequence of the human CD1e heavy chain can be found in Uniprot accession number P15812.

In the present application, the heavy chain of the MHC-like molecule may be a MR1 heavy chain. In the present application, the presenting peptide can form a fusion protein with the B2M protein, and the fusion protein can bind to the MR1 heavy chain to form a complex that binds to inhibitory receptors of immune cells. In the complex, the presenting peptide can bind to the peptide binding groove of the MR1 heavy chain. In the present application, the polynucleotide inserted into the cell may not comprise a nucleic acid molecule encoding the MR1 heavy chain. For example, the amino acid sequence of the human MR1 heavy chain can be found in Uniprot accession number Q95460.

In the present application, the heavy chain of the MHC-like molecule may be a FcRn heavy chain. In the present application, the presenting peptide can form a fusion protein with the B2M protein, and the fusion protein can bind to the FcRn heavy chain to form a complex that binds to inhibitory receptors of immune cells. In the complex, the presenting peptide can bind to the peptide binding groove of the FcRn heavy chain. In the present application, the polynucleotide inserted into the cell may not comprise a nucleic acid molecule encoding the FcRn heavy chain. For example, the amino acid sequence of the human FcRn heavy chain can be found in Uniprot accession number P55899.

In the present application, the heavy chain of the MHC-like molecule may be a UL18 heavy chain. In the present application, the presenting peptide can form a fusion protein with the B2M protein, and the fusion protein can bind to the UL18 heavy chain to form a complex that binds to inhibitory receptors of immune cells. In the complex, the presenting peptide can bind to the peptide binding groove of the UL18 heavy chain. In the present application, the polynucleotide inserted into the cell may not comprise a nucleic acid molecule encoding the UL18 heavy chain. For example, the amino acid sequence of the human UL18 heavy chain can be found in Uniprot accession number Q0QIE0.

### Inhibitory receptor

In the present application, the complex formed by the fusion protein of B2M and the presenting peptide as well as the heavy chain of the MHC molecule can bind to inhibitory receptors on the surface of immune cells. For example, the inhibitory receptor may be an inhibitory receptor of a T cell or an inhibitory receptor of an NK cell. For example, the inhibitory receptor may be one or more selected from the group comprising: NKG2A/KLRD1 (CD159a/CD94), NKG2B, KIR2DL1 (CD158a), KIR2DL2 (CD158b), KIR2DL3 (CD158d), KIR2DL5 (CD158f), KIR3DL1 (CD158e1), KIR3DL2 (CD158k), KIR3DL3, ILT2/LIR-1 (CD85J), LAG-3 (CD223) and/or KIR2DL4 (CD158d).

In the present application, the inhibitory receptor of the immune cell may be NKG2A. The presenting peptide can form a fusion protein with the B2M protein, and the fusion protein can form a complex with the heavy chain of the MHC molecule. The complex can bind to the NKG2A receptor of an immune cell, thereby inhibiting immune cell activation. For example, the immune cell can be a T cell. For example, the immune cell can be a NK cell. The amino acid sequence of the human NKG2A can be found in Uniprot accession number P26715.

In the present application, the inhibitory receptor of the immune cell may be a KIR receptor. For example, the KIR receptor may be selected from KIR2DL1, KIR2DL2, KIR2DL3, KIR2DL4, KIR2DL5, KIR3DL1, KIR3DL2, and KIR3DL3.

In the present application, the inhibitory receptor of the immune cell is KIR2DL1. The presenting peptide can form a fusion protein with the B2M protein, and the fusion protein can form a complex with the heavy chain of the MHC molecule. The complex can bind to the KIR2DL1 receptor of an immune cell, thereby inhibiting immune cell activation. For example, the immune cell can be a T cell. For example, the immune cell can be a NK cell. The amino acid sequence of the human KIR2DL1 can be found in Uniprot accession number A0A191URJ7.

In the present application, the inhibitory receptor of the immune cell is KIR2DL2. The presenting peptide can form a fusion protein with the B2M protein, and the fusion protein can form a complex with the heavy chain of the MHC molecule. The complex can bind to the KIR2DL2 receptor of an immune cell, thereby inhibiting immune cell activation. For example, the immune cell can be a T cell. For example, the immune cell can be a NK cell. The amino acid sequence of the human KIR2DL2 can be found in Uniprot accession number Q6H2H0.

In the present application, the inhibitory receptor of the immune cell is KIR2DL3. The presenting peptide can form a fusion protein with the B2M protein, and the fusion protein can form a complex with the heavy chain of the MHC molecule. The complex can bind to the KIR2DL3 receptor of an immune cell, thereby inhibiting immune cell activation. For example, the immune cell can be a T cell. For example, the immune cell can be a NK cell. The amino acid sequence of the human KIR2DL3 can be found in Uniprot accession number E3NZD8.

In the present application, the inhibitory receptor of the immune cell is KIR2DL4. The presenting peptide can form a fusion protein with the B2M protein, and the fusion protein can form a complex with the heavy chain of the MHC molecule. The complex can bind to the KIR2DL4 receptor of an immune cell, thereby inhibiting immune cell activation. For example, the immune cell can be a T cell. For example, the immune cell can be a NK cell. The amino acid sequence of the human KIR2DL4 can be found in Uniprot accession number Q99706.

In the present application, the inhibitory receptor of the immune cell is KIR2DL5. The presenting peptide can form a fusion protein with the B2M protein, and the fusion protein can form a complex with the heavy chain of the MHC molecule. The complex can bind to the KIR2DL5 receptor of an immune cell, thereby inhibiting immune cell activation. For example, the immune cell can be a T cell. For example, the immune cell can be a NK cell. The amino acid sequence of the human KIR2DL5 can be found in Uniprot accession number B0L653.

In the present application, the inhibitory receptor of the immune cell is KIR3DL1. The presenting peptide can form a fusion protein with the B2M protein, and the fusion protein can form a complex with the heavy chain of the MHC molecule. The complex can bind to the KIR3DL1 receptor of an immune cell, thereby inhibiting immune cell activation. For example, the immune cell can be a T cell. For example, the immune cell can be a NK cell. The amino acid sequence of the human KIR3DL1 can be found in Uniprot accession number P43629.

In the present application, the inhibitory receptor of the immune cell is KIR3DL2. The presenting peptide can form a fusion protein with the B2M protein, and the fusion protein can form a complex with the heavy chain of the MHC molecule. The complex can bind to the KIR3DL2 receptor of an immune cell, thereby inhibiting immune cell activation. For example, the immune cell can be a T cell. For example, the immune cell can be a NK cell. The amino acid sequence of the human KIR3DL2 can be found in Uniprot accession number P43630.

In the present application, the inhibitory receptor of the immune cell is KIR3DL3. The presenting peptide can form a fusion protein with the B2M protein, and the fusion protein can form a complex with the heavy chain of the MHC molecule. The complex can bind to the KIR3DL3 receptor of an immune cell, thereby inhibiting immune cell activation. For example, the immune cell can be a T cell. For example, the immune cell can be a NK cell. The amino acid sequence of the human KIR3DL3 can be found in Uniprot accession number Q8N743.

In the present application, the inhibitory receptor of the immune cell is leukocyte immunoglobulin-like receptor 1 "LIR1". The presenting peptide can form a fusion protein with the B2M protein, and the fusion protein can form a complex with the heavy chain of the MHC molecule. The complex can bind to the LIR1 receptor of an immune cell, thereby inhibiting immune cell activation. For example, the immune cell can be a T cell. For example, the immune cell can be a NK cell. The amino acid sequence of the human LIR1 can be found in Uniprot accession number Q96500.

### Presenting peptide

In the present application, the presenting peptide can form a fusion protein with the B2M protein, and the fusion protein can bind to the heavy chain of an MHC molecule to form a complex that binds to inhibitory receptors of immune cells. In the complex, the presenting peptide can bind to the peptide binding groove of the heavy chain of the MHC molecule.

In the present application, the presenting peptide may be derived from a virus, a prokaryote, an eukaryote or a mammal. For example, the presenting peptide may be derived from a virus. For example, the presenting peptide may be derived from a prokaryote. For example, the presenting peptide may be derived from an eukaryote. For example, the presenting peptide may be derived from a mammal. For example, the presenting peptide may be derived from a human. The presenting peptide may have 5-30, 5-25, 5-20, 7-20, 7-18, 7-15, 8-12 or 8-10 amino acids. For example, the presenting peptide may have 5-30 amino acids. For example, the presenting peptide may have 5-25 amino acids. For example, the presenting peptide may have 5-20 amino acids. For example, the presenting peptide may have 7-20 amino acids. For example, the presenting peptide may have 7-18 amino acids. For example, the presenting peptide may have 7-15 amino acids. For example, the presenting peptide may have 8-12 amino acids. For example, the presenting peptide may have 8-10 amino acids.

In the present application, the presenting peptide may be derived from a viral protein, such as CMV, EBV, and HIV. In some embodiments, the presenting peptide is a fragment of CMV. In some embodiments, the presenting peptide is a fragment of EBV. In some embodiments, the presenting peptide is a fragment of HIV. For example, the presenting peptide may be derived from an ATP binding cassette transporter. For example, the presenting peptide may be derived from multidrug resistance-associated protein 7. For example, the presenting peptide may be derived from gliadin. For example, the presenting peptide may be derived from a HSP60 conserved region. For example, the presenting peptide may be derived from a GroEL conserved region.

In the present application, the presenting peptide may be derived from a signal peptide of a MHC class I molecule or a fragment thereof. In the present application, the presenting peptide may be a fragment of a signal peptide of a MHC class I molecule. The signal peptide of the MHC class I molecule may have 13-80 amino acids. The signal peptide of the MHC class I molecule may have 20-50 amino acids.

In the present application, the presenting peptide may be an HLA-E restricted presenting peptide. In the present application, the presenting peptide may be derived from a signal peptide of a MHC class I molecule or a fragment thereof. In the present application, the presenting peptide may be derived from a signal peptide of an HLA-A molecule or a fragment thereof. In the present application, the presenting peptide may be derived from a signal peptide of an HLA-B molecule or a fragment thereof. In the present application, the presenting peptide may be derived from a signal peptide of an HLA-C molecule or a fragment thereof. In the present application, the presenting peptide may be derived from a signal peptide of an HLA-G molecule or a fragment thereof. In the present application, the presenting peptide may be derived from a signal peptide of an HLA-F molecule or a fragment thereof. In the present application, the presenting peptide may be derived from a signal peptide selected from the following proteins or fragments thereof: HLA-Al, HLA-A2, HLA-A*02, HLA-A*0203, HLA-A*0210, HLA-A3, HLA-A*23, HLA-A*24, HLA-A*2403, HLA-A*2410, HLA-A*2502, HLA-A*2501, HLA-A*26, HLA-A*66(01,02), HLA-A9, HLA-A*6603, HLA-A10, HLA-A*11, HLA-A19, HLA-A*29, HLA-A*30, HLA-A*31, HLA-A*32, HLA-A*33, HLA-A*74, HLA-A*7403, HLA-A28, HLA-A36, HLA-A34, HLA-A43, HLA-A*3401, HLA-A*80, HLA-B5, HLA-B7, HLA-B8, HLA-B12, HLA-B13, HLA-B14, HLA-B15, HLA-B16, HLA-B17, HLA-B18, HLA-B21, HLA-B22, HLA-B27, HLA-B35, HLA-B37, HLA-B38, HLA-B39, HLA-B40, HLA-B41, HLA-B42, HLA-B46, HLA-B47, HLA-B48, HLA-B49, HLA-B50, HLA-B51, HLA-B52, HLA-B53, HLA-B54, HLA-B55, HLA-B56, HLA-B57, HLA-B58, HLA-B59, HLA-B60, HLA-B61, HLA-B62, HLA-B63, HLA-B64, HLA-B65, HLA-B67, HLA-B70, HLA-B71, HLA-B73, HLA-B75, HLA-B76, HLA-B77, HLA-B78, HLA-B81, HLA-B82, HLA-B83, HLA-Cw1, HLA-Cw2, HLA-Cw3, HLA-Cw4, HLA-Cw5, HLA-Cw6, HLA-Cw7, HLA-Cw8, HLA-Cw9, HLA-Cw10, HLA-Cw*0809, HLA-Cw7, HLA-Cw*1701, Cwx, HLA-G and HLA-F.

The presenting peptide that is a signal peptide of a MHC class I molecule or a fragment thereof may have 5-30 amino acids. For example, the presenting peptide can be a signal peptide of a MHC class I molecule or a fragment thereof, and can have 5-25 amino acids, 5-20 amino acids, 7-18 amino acids, 7-15 amino acids, 8 amino acids, 12 amino acids or 8-10 amino acids. For example, the presenting peptide may be a fragment of a signal peptide of a MHC class I molecule, or a fragment thereof, and may have 8-10 amino acids.

For example, the presenting peptide may be a signal peptide of a MHC class I molecule or a fragment thereof, and have the following amino acid sequence: X₁X₂X₃X₄X₅X₆X₇X₈L, where X₁ is V or I; X₂ is T, A, M or L; X₃ is A, P, K or N; X₄ is P, L or T; X₅ is R, Q or K; X₆ is T or A; X₇ is L, I, V or P; X₈ is V, L, I, F or T (SEQ ID NO: 17).

For example, the presenting peptide may be derived from a signal peptide of a MHC class I molecule, or a fragment thereof, and the amino acid sequence may comprise an amino acid sequence as shown in any one of SEQ ID NOs: 18-70. For example, the presenting peptide may comprise an amino acid sequence as shown in SEQ ID NO: 35, SEQ ID NO: 30 or SEQ ID NO: 33.

### Linker

In the present application, the polynucleotide may further comprise a nucleic acid molecule encoding a linker. The presenting peptide can be covalently linked to the B2M protein through a linker. The linker can link the presenting peptide and the B2M protein and provide sufficient flexibility so that the fusion protein of the presenting peptide and the B2M fusion protein can form a stable complex with the heavy chain of the MHC molecule. For example, the linker can be located at the C-end of the presenting peptide. For example, after the expression of the modified cell on surface, the presenting peptide, the linker and the B2M protein can be linked sequentially from the N-terminus to the C-terminus. For example, the linker can be located at the N-end of the presenting peptide. For example, after the expression of the modified cell on surface, the B2M, the linker and the presenting peptide protein can be linked sequentially from the N-terminus to the C-terminus.

In the present application, the linker may have about 5 to 50 amino acids and include at least one of the following five amino acids: Gly, Ala, Pro, Val and Leu, and at least one of the eight amino acids: Ser, Thr, Glu, Lys, Asn, Gln, Asp and Arg. For example, the linker may have 5 to 30 amino acids.

In the present application, the amino acid sequence of the linker may be (EAAAK)ₙ, where n = 1, 2, 3, 4 or 5. In the present application, the amino acid sequence of the linker may be (EAAAK)ₙ, where n = 3, 4 or 5. In the present application, the amino acid sequence of the linker may be (GGGGS)ₙ, where n = 1, 2, 3, 4 or 5. In the present application, the amino acid sequence of the linker may be (GGGGS)ₙ, where n = 3, 4 or 5.

In the present application, the amino acid sequence of the linker is (G)nS, where n = 1, 2, 3, 4 or 5. In the present application, the amino acid sequence of the linker is ((G)ₙS)ₘ, where n = 1, 2, 3, 4 or 5, m = 1 , 2, 3, 4 or 5. m = 1, 2, 3, 4 or 5. For example, the amino acid sequence of the linker may be as shown in any one of SEQ ID NO: 71-99.

In the present application, the polynucleotide may comprise a nucleic acid molecule encoding the presenting peptide. In the present application, the polynucleotide may comprise a nucleic acid molecule encoding the presenting peptide and a nucleic acid molecule encoding the linker. In the present application, the polynucleotide may comprise a nucleic acid molecule encoding the presenting peptide, a nucleic acid molecule encoding the linker, and a nucleic acid molecule encoding the B2M protein fragment.

For example, the polynucleotide may encode an amino acid sequence as shown in SEQ ID NO: 9. For example, the polynucleotide may encode an amino acid sequence that is at least 80%, 85%, 90%, 99.1%, 99.2%, 99.3%, 99.4%, 99.5%, 99.6%, 99.7%, 99.8% or 99.9% identical to the amino acid sequence as shown in SEQ ID NO: 9. For example, the polynucleotide may comprise a nucleotide sequence as shown in SEQ ID NO: 12. For example, the polynucleotide may comprise a nucleotide sequence that is at least 80%, 85%, 90%, 99.1%, 99.2%, 99.3%, 99.4%, 99.5%, 99.6%, 99.7%, 99.8% or 99.9% identical to the nucleotide sequence as shown in SEQ ID NO: 12.

### Cell

In another aspect, the present application provides a modified immune cell. For example, the expression of a classic MHC class I molecule on the cell surface of the modified cell may be down-regulated (e.g., compared to immune cells without the modification, down-regulated by at least 10%, at least 20%, at least 30%, at least 40%, at least 50%, at least 60%, at least 70%, at least 80%, at least 90% or more), and the expression of a non-classical MHC class I molecule may be positive. For example, the expression of a classic MHC class I molecule on the cell surface of the modified cell may be down-regulated (e.g., compared to immune cells without the modification, down-regulated by at least 10%, at least 20%, at least 30%, at least 40%, at least 50%, at least 60%, at least 70%, at least 80%, at least 90% or more), and the expression of a non-classical MHC class I molecule may substantially not be down-regulated. For example, the expression of a classic MHC class I molecule on the cell surface of the modified cell may be down-regulated (e.g., compared to immune cells without the modification, down-regulated by at least 10%, at least 20%, at least 30%, at least 40%, at least 50%, at least 60%, at least 70%, at least 80%, at least 90% or more), and the expression of a non-classical MHC class I molecule may be up-regulated (e.g., compared to immune cells without the modification, up-regulated by at least 10%, at least 20%, at least 30%, at least 40%, at least 50%, at least 60%, at least 70%, at least 80%, at least 90% or more).

In the present application, the modified immune cell may comprise a T cell receptor α constant region protein (TRAC), a T cell receptor β constant region protein (TRBC), CD3ε, CD3y, CD3δ and/or CD3ζ whose expression and/or activity are down-regulated.

In the present application, the modified cell may comprise a gene encoding a chimeric antigen receptor (CAR), a T cell receptor (TCR), a chimeric autoantibody receptor (CAAR), a TCR receptor fusion construct (TRuC), a T cell antigen coupler (TAC), an antibody TCR receptor (AbTCR), a chimeric CD3ε receptor and/or at least one other synthetic receptor.

In the present application, the modified cell may express a chimeric antigen receptor (CAR), a T cell receptor (TCR), a chimeric autoantibody receptor (CAAR), a TCR receptor fusion construct (TRuC), a T cell antigen coupler (TAC), an antibody TCR receptor (AbTCR), a chimeric CD3ε receptor and/or at least one other synthetic receptor.

In the present application, the modified cell may be an immune cell. The immune cell may comprise a T cell, a B cell, a natural killer (NK) cell, a macrophage, an NKT cell, a monocyte, a dendritic cell, a granulocyte, a lymphocyte, a leukocyte, and/or a peripheral blood mononuclear cell. In certain cases, the immune cell may comprise a T lymphocyte. The T lymphocyte may comprise a thymocyte, an innate T lymphocyte, an immature T lymphocyte, a mature T lymphocyte, a resting T lymphocyte, or an activated T lymphocyte. The T cell can be a helper T cell (Th), for example, a helper T cell 1 (Th1) or a helper T cell 2 (Th2). The T lymphocyte may be a CD4+ helper T cell (HTL; a CD4⁺ T cell), a cytotoxic T cell (CTL; a CD8⁺ T cell), a tumor-infiltrating cytotoxic T cell (TIL; a CD8⁺ T cell), a CD4+/CD8⁺ T cell, a CD4-/CD8⁻ T cell, or any other T lymphocyte subtype. In certain cases, the modified cell is a human T cell.

In certain cases, the immune cell may comprise a B cell. In certain cases, the B cell may comprise an effector B cell (plasma cell) and a memory B cell. The B cell may comprise a B2 cell, a B1 cell, a marginal zone B cell, a follicular B cell, and a regulatory B cell. In certain cases, the immune cell may comprise a macrophage. The B cell may comprise a type I macrophage (M1) and a type II macrophage (such as M2a, M2B and M2c). In certain cases, the immune cell may comprise an NK cell. In certain cases, the NK cell may comprise CD56bright and CD56dim. In certain cases, the NK cell may comprise NK1 and NK2. In certain cases, the NK cell may comprise A-NK and NA-NK.

The modified immune cell of the present application may be activated and proliferated before or after any modification step. The immune cell may be proliferated *in vitro* or *in vivo.*

Prior to proliferation and genetic modification of the cells of the present application, cells can be obtained from a subject (e.g., a patient) through a variety of non-limiting methods. The immune cell can be obtained from a number of non-limiting sources, including peripheral blood mononuclear cells, bone marrow, lymph node tissue, umbilical cord blood, thymus tissue, tissue from a site of infection, ascites, pleural effusion, spleen tissue, and tumors. In certain cases, any number of immune cell lines, available and known to a person skilled in the art, can be used. In another case, the cell can be derived from a healthy donor, from a patient diagnosed with cancer or from a patient diagnosed with an infection. In another case, the cell is a portion of a mixed cell population which present different phenotypic characteristics. In certain cases, the immune cell may be an autologous cell derived from a subject. In certain cases, the immune cell may be derived from an allogeneic cell, such as from a donor compatible with the human leukocyte antigen (HLA) of the subject.

### Preparation method

In another aspect, the present application provides a method for preparing a modified cell, the method comprises inserting the polynucleotide encoding the presenting peptide into the cell by introducing a vector comprising the polynucleotide. For example, the inserting may comprise contacting a vector comprising the polynucleotide with the cell. For example, the vector comprising the polynucleotide may not comprise a complete B2M gene coding region. For example, the vector comprising the polynucleotide may not comprise a nucleic acid molecule encoding the heavy chain of the MHC molecule. For example, the vector comprising the polynucleotide may further comprises a homology arm. For example, the vector comprising the polynucleotide may comprise an upstream homology arm, the polynucleotide encoding the presenting peptide and a downstream homology arm.

For example, the vector comprising the polynucleotide may comprise a nucleotide sequence as shown in SEQ ID NO: 4. For example, the vector comprising the polynucleotide may comprise a nucleotide sequence that is at least 80%, 85%, 90%, 99.1%, 99.2%, 99.3%, 99.4%, 99.5%, 99.6%, 99.7%, 99.8% or 99.9% identical to the nucleotide sequence as shown in SEQ ID NO: 4.

The modification can be performed using gene editing technology based on the CRISPR system. In the present application, the method comprises administering a CRISPR/Cas system to the immune cell, and the system may include an RNA component, sometimes referred to as guide RNA (gRNA).

The guide RNA described in the present application may comprise single-stranded guide RNA (sgRNA) as well as double-stranded guide RNA composed of crRNA (CRISPR RNA) and tracrRNA (trans-activating crRNA). In certain embodiments, the guide RNA has a double-stranded structure consisting of a crRNA and a tracrRNA. In certain embodiments, the guide RNA is a single-stranded molecule that may comprise a guide sequence, a tracr mate sequence, and a tracr sequence.

In certain cases, the guide RNA described in the present application may be complementary to a target nucleic acid (for example, the endogenous B2M gene). In other cases, the guide RNA may be identical to the target nucleic acid (and when speaking of identical, the "U" in RNA corresponds to the thymine "T" in DNA due to the difference in the bases encoding RNA and DNA). In other cases, the nucleic acid sequence (for example, DNA) encoding the guide RNA may be identical to or complementary to the target nucleic acid. The guide RNA can be obtained by transcription or replication of the sequence encoding it. For example, the guide RNA can be obtained by transcription of the DNA sequence encoding it. In some cases, the percent complementarity between the guide RNA and the target nucleic acid (for example, the endogenous B2M gene) may be at least about 30%, at least about 40%, at least about 50%, at least about 60%, at least about 65%, at least about 70%, at least about 75%, at least about 80%, at least about 85%, at least about 90%, at least about 95%, at least about 97%, at least about 98%, at least about 99%, or 100%. In some cases, the percent complementarity between the guide RNA and the target nucleic acid (for example, the endogenous B2M gene) may be at most about 30%, at most about 40%, at most about 50%, at most about 60%, at most about 65%, at most about 70%, at most about 75%, at most about 80%, at most about 85%, at most about 90%, at most about 95%, at most about 97%, at most about 98%, at most about 99%, or 100%.

The method of the present application comprises providing a nucleic acid targeting the genome of an immune cell (for example, the endogenous B2M gene), and the nucleic acid can guide a relevant active polypeptide (for example, a Cas protein) into a specific target sequence within a target nucleic acid (for example, the endogenous B2M gene). The nucleic acid targeting the genome may be RNA.

In the present application, the guide RNA of the nucleic acid molecule targeting the endogenous B2M gene may comprise a nucleotide sequence as shown in SEQ ID NO: 13. For example, the guide RNA of the nucleic acid molecule targeting the endogenous B2M gene may comprise a nucleotide sequence having at least 80% (e.g., at least 85%, at least 90%, at least 91%, at least 92%, at least 93%, at least 94%, at least 95%, at least 96%, at least 97%, at least 98%, at least 99%, or at least 100%) sequence identity to the nucleotide sequence as shown in SEQ ID NO: 13.

The method may further comprise down-regulating the expression and/or activity of a T cell receptor α constant region protein (TRAC), a T cell receptor β constant region protein (TRBC), CD3ε, CD3y, CD3δ and/or CD3ζ. For example, the method may comprise administering a guide RNA targeting a nucleic acid molecule encoding the TRAC to the cell. In the present application, the guide RNA of the nucleic acid molecule targeting the TRAC gene may comprise a nucleotide sequence as shown in SEQ ID NO: 7. For example, the guide RNA of the nucleic acid molecule targeting the endogenous B2M gene may comprise a nucleotide sequence having at least 80% (e.g., at least 85%, at least 90%, at least 91%, at least 92%, at least 93%, at least 94%, at least 95%, at least 96%, at least 97%, at least 98%, at least 99%, or at least 100%) sequence identity to the nucleotide sequence as shown in SEQ ID NO: 7.

In certain cases, the guide RNA may comprise a backbone sequence that does not affect the recognition of the target sequence by the sgRNA. Therefore, the backbone sequence may be any feasible sequence in the prior art. The backbone sequence generally comprises a tracr mate sequence and a tracr sequence. For example, the backbone sequence may comprise a nucleotide sequence as shown in SEQ ID NO: 2.

The guide RNA of the present application may further comprise a modification (for example, a chemical modification), such as deletion, insertion, translocation, inactivation and/or activation of a nucleotide. Such a modification may comprise introducing of one or more mutations (comprising single or multiple base pair changes), increasing the number of hairpins, cross-linking, breaking a specific stretch of nucleotides, and other modifications.

In certain embodiments, the method described in the present application may comprise administering one or more of the Cas proteins to a host cell (for example, an immune cell).

In another aspect, the present application provides a CRISPR enzyme. In some cases, the CRISPR enzyme may be a Type II CRISPR system enzyme. In certain cases, the CRISPR enzyme may be a Cas9 protein. In certain embodiments, the Cas9 protein may be a *Streptococcus pneumoniae, Streptococcus pyogenes,* or *Streptococcus thermophilus* Cas9 protein, and may comprise a mutated Cas9 protein derived from these organisms. The Cas protein may also be a homologue or ortholog of a Cas9 protein. The system described in this application may comprise the Cas protein. The Cas protein may comprise any other protein, and optionally a linking sequence between any two domains. Examples of proteins that can be fused to the Cas protein comprise, but are not limited to, epitope tags, reporter genes, and protein domains with one or more of the following activities: methylase activity, demethylase activity, transcription activation activity, transcription repression activity, transcription release factor activity, histone modification activity, RNA cleavage activity and nucleic acid binding activity.

The CRISPR system of the present application can be transferred into an immune cell by a method known in the art, such as: calcium phosphate transfection, protoplasm fusion, electroporation, lipofectamine transfection, microinjection, viral infection (such as baculovirus, vaccinia virus, adenovirus and other viruses), *etc.* For example, the CRISPR system (for example, Cas protein and/or guide RNA) can be introduced into an immune cell using a electroporation method. For the electrotransformation method, please refer to the literature Schumann et al. PNAS 2015.112:10437-10442.

In another aspect, the present application provides a nucleic acid combination comprising the isolated nucleic acid molecule and the guide RNA in the method.

In another aspect, the present application provides a vector comprising the isolated nucleic acid molecule and/or the nucleic acid combination. In certain embodiments, the vector is a viral vector. In certain embodiments, the vector is a lentiviral vector, an adenoviral vector, or an adeno-associated viral vector.

In another aspect, the present application provides a host cell comprising the isolated nucleic acid molecule, the guide RNA, the nucleic acid combination and/or the vector.

In another aspect, the present application provides a cell population comprising at least 30% of the modified cell.

In another aspect, the present application provides a pharmaceutical composition comprising the isolated nucleic acid molecule, the guide RNA, the nucleic acid combination, the vector, the modified cell, the host cell and/or the cell population, and optionally a pharmaceutically acceptable carrier. The "pharmaceutically acceptable carrier" described in the present application may comprise any and all solvents, dispersion media, coatings, antibacterial and antifungal agents, isotonic agents and absorption delaying agents and the like that are physiologically compatible. In certain instances, the carrier is suitable for intravenous, intramuscular, subcutaneous, parenteral, spinal or epidermal administration (e.g. by injection or infusion). The "effective amount" generally refers to an amount of a substance, compound, material or cell that is at least sufficient to produce a therapeutic effect when administered to a subject. Thus, it is an amount essential for preventing, curing, ameliorating, blocking or partially blocking the symptoms of a disease or condition.

In another aspect, the present application provides the use of the isolated nucleic acid molecule, the guide RNA, the nucleic acid combination, the vector, the modified cell, the cell, the cell population and/or the pharmaceutical composition in the preparation of a medicine for allogeneic transplantation.

In another aspect, the present application provides a method for allogeneic transplantation, the method comprises administering the isolated nucleic acid molecule, the guide RNA, the nucleic acid combination, the vector, the modified cell, the cell, the cell population and/or the pharmaceutical composition to a subject.

In another aspect, the present application provides a method for increasing allogeneic transplantation compatibility, the method comprises using the method for preparing a modified cell.

In another aspect, the present application provides the use of the isolated nucleic acid molecule, the guide RNA, the nucleic acid combination, the vector, the modified cell, the cell, the cell population and/or the pharmaceutical composition in the preparation of a medicine for the treatment or alleviation of a tumor.

In another aspect, the present application provides a method for treating or alleviating a tumor, the method comprises administering the isolated nucleic acid molecule, the guide RNA, the nucleic acid combination, the vector, the modified cell, the cell, the cell population and/or the pharmaceutical composition to a subject.

Without intending to be limited by any theory, the following examples are only for illustrating various technical solutions of the invention of the present application, but are not intended to limit the scope of the invention of the present application.

### Example

### Example 1 Design of gRNA targeting B2M

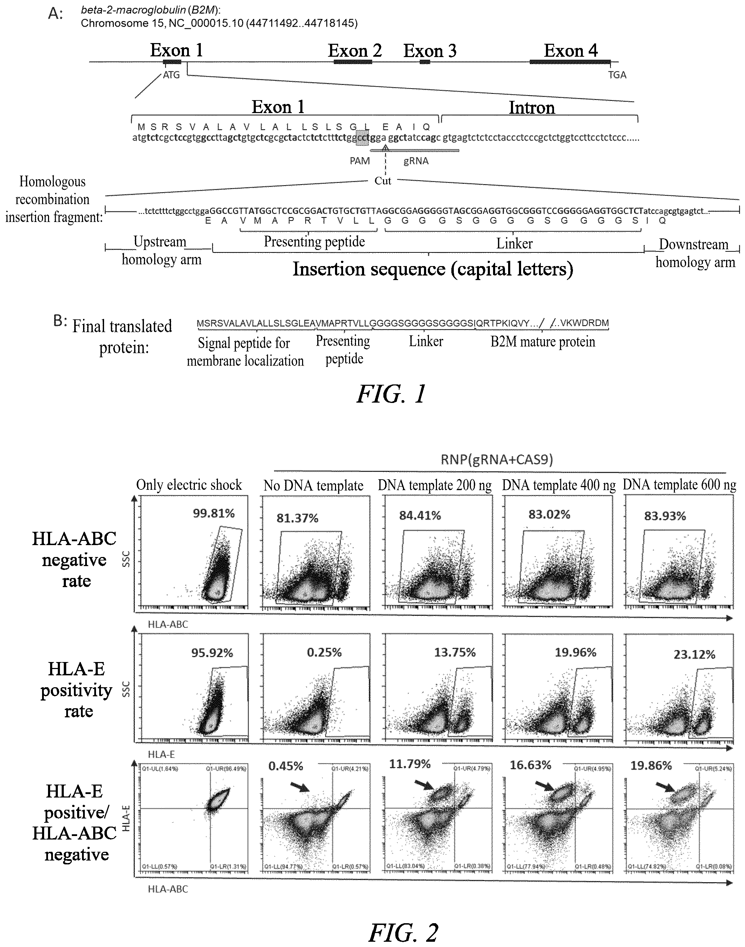

CRISPR/Cas9-mediated gene editing is used to achieve targeted knock-in of the HLA-E restricted peptide into the B2M protein, so that the new protein structure formed after the targeted knock-in is "signal peptide - presenting peptide - linker - B2M mature protein ", and an exemplary final translated protein structure is shown in Fig. 1B.

In order to design a guide RNA (gRNA) to achieve efficient and accurate targeted knock-in, we searched the coding exon 1 and exon 2 of the *B2M* gene, the location of NGG binding, the location of the target sequence insertion and the sequences of the homology arms at both ends. After comprehensive analysis, the sequence of the gRNA was determined, and an exemplary gRNA sequence was shown in Fig. 1A and targeted the antisense strand of exon 1 of the *B2M* gene, and the targeted sequence was SEQ ID NO:1 according to the direction of 5'-3': CTCACGCTGGATAGCCTCC (SEQ ID NO: 1).

### Example 2 Preparation of CRISPR/Cas9 RNP + DNA homologous recombination template system

Source of Cas9 protein: The type was derived from wild type of *Streptococcus pyogenes* (SpCas9), had a nuclear localization signal (NLS), and was purchased from Integrated DNA Technologies (catalog number: 1074181).

gRNA source: The CRISPR/Cas9 system in the example was derived from *Streptococcus pyogenes.* The single-stranded guide RNA (sgRNA) was composed of two parts. The first part at the 5' end contains a 19-base guide sequence corresponding to the target sequence (the guide sequence is the same as the 19 bases immediately before the 5' end of PAM, but is an RNA chain, that is, T in the nucleotide sequence as shown in SEQ ID NO: 1 was replaced by U, and the nucleotide sequence of the guide sequence obtained was shown in SEQ ID NO: 14), the backbone sequence linking the second part was: 5'GUUUUAGAGCUAGAAAUAGCAAGUUAAAAUAAGGCUAGUCCGUUAUCAAC UUGAAAAAGUGGCACCGAGUCGGUGCUUUU 3' (SEQ ID NO: 2). The number of base U at the end of the backbone sequence can vary. The sgRNA sequence used in this example was SEQ ID NO: 3: 
5'cucacgcuggauagccuccGUUUUAGAGCUAUGCUGGAAACAGCAUAGCAAGUUAAAA
UAAGGCUAGUCCGUUAUCAACUUGAAAAAGUGGCACCGAGUCGGUGCUUUU 3'
 Among them, lowercase letters represented the gRNA, and uppercase letters represented the backbone sequence. The sequence was synthesized by Nanjing GenScript Biotechnology Co., Ltd.

Source of homologous recombination DNA: The homologous recombination insertion fragment employed a double-stranded DNA (dsDNA) amplified by PCR, or employed an adeno-associated virus (AAV) to deliver the DNA template. The homologous recombination fragment comprised upstream homology arm-insertion sequence-downstream homology arm, as shown in Fig. 1A. The insertion sequence comprised the HLA-E restricted presenting peptide and the linker. The nucleotide sequence of the insertion sequence was as shown in SEQ ID NO: 4. The amino acid sequence of the protein expressed by the insertion sequence was as shown in SEQ ID NO: 9. After the gene was fully synthesized, it was used as a PCR template, and the PCR primers were as follows:

**Table 1. PCR primers for amplifying homologous recombination DNA**

| Primer name | Sequence |
|---|---|
| Upstream F: | 5'ATCTTAATCTTCTGGGTTTCCG3' (SEQ ID NO: 11) |
| Downstream R: | 5'TCTAAGGGAAGCAGAGCCGCAG3' (SEQ ID NO: 12) |

For PCR amplification reaction, the polymerase was KOD FX Neo (TOYOBO, cat#636200). The reaction conditions were as follows in Table 2:

**Table 2. PCR reaction conditions for amplifying homologous recombination**

| | |
|---|---|
| 1 | 95°C 3 min |
| 2 | 95°C 20 s |
| 3 | 58°C 20 s |
| 4 | 68°C 50 s |
| 5 | 72°C 2 min |
| 6 | 2-5 were cycled for 32 times. |
| 7 | Stored at 4°C |

### Example 3 Targeted knock-in of HLA-E restricted presenting peptide at the B2M locus in primary T cells

Primary T cells were derived from peripheral blood mononuclear cells (PBMC) from healthy human donors. T cell culture medium was complete culture medium: ImmunoCult^{™}-XF T Cell Expansion Medium (Stem Cell Technology, cat#10981) plus 300 IU/ml IL2 (Cayan, cat# HEILP-0201c).

### PBMC activation and culture:

(1) Counting the PBMC after resuscitation: the PBMC cell suspension was pipetted 5-6 times with a 1 ml pipette and mixed thoroughly. 20 µl of the resulting cell suspension was take out, and 180 µl of PBS (Gibco, cat#C10010500BT) was added, and the cell counter NC200 was used for counting.
(2) 5E6 cells were taken and CD3/CD28 activated magnetic beads Dynabeads (Thermo, cat#11141D) at a ratio of 1:3 were added, that is, 1.5E7 magnetic beads were required. After thorough mixing, the complete culture medium was added to a 6-well cell culture plate with a cell culture density of 1. 5E6/ml, that is, the volume of the culture medium was 3.3 ml, and the plate was placed in a 37-degree carbon dioxide incubator for culture.
(3) On day 3 of culture, the magnetic beads were removed.
(4) On day 5 of culture, and the gene editing experiment with targeted insertion was carried out.

### CRISPR/Cas9-mediated gene-targeted insertion:

(1) Preparing cells: cells for electrotransformation were washed 2 times with opti-MEM medium (Gibco, cat#31985-070), counted and adjusted to the concentration to 3.4E7/ml, and 6 ul was taken for electroporation (about 2E5 cells);
(2) Preparing sgRNA and Cas9 protein: the concentration of the sgRNA was adjusted to 200 ng/ul with ddH2O, and 2 µL was used for electrotransformation; the concentration of the Cas9 protein was adjusted to 500 ng/µl with opti-MEM, and 2 µL was taken for electrotransformation; the concentration of the homologous recombinant DNA was adjusted to 100 ng/µl with ddH2O, and 2 µL was used for electrotransformation; firstly the sgRNA and the Cas9 were mixed together, incubated at room temperature for 10 minutes, and then mixed with the homologous recombination DNA and the cells; the volume was made up to 10 µl with opti-MEM medium;
(3) Electrotransformation: Neon transfection system (Thermo Fisher Scientific) was used for electrotransformation. Electrotransformation parameters: 1500 V, 10 ms, 3 times. After electrotransformation, the cells were added to 200 µL of complete culture medium and cultured in a 96U-type plate.
(4) The design of each group was shown in Table 3.

**Table 3. Design of CRISPR/Cas9-mediated gene-targeted insertion experiment**

| Number | RNP (sgRNA+Cas9) | Homologous recombination DNA | Notes |
|---|---|---|---|
| 1 | - | - | Control group - only electrotransformation |
| 2 | + | - | Control group - only knock-out without knock-in |
| 3 | + | 200 ng | Gradiently increasing accumulation of homologous recombinant DNA for gene knock-in |
| 4 | + | 400 ng | |
| 5 | + | 600 ng | |

### Example 4 Detection of targeted insertion efficiency in primary T cells (protein level detection by flow cytometry)

B2M is a small subunit in the HLA-ABC and HLA-E complex that combines with the HLA-A/B/C heavy chains, the HLA-E heavy chain, or the heavy chain of other HLA molecules to form a complex. The normal expression of B2M ensures the normal expression of HLA-ABC and HLA-E complex. When B2M loses its function, cells cannot express HLA-ABC or HLA-E. After being cleaved by CRISPR/Cas9, cells would repair DNA damage through two pathways, non-homologous end joining (NHEJ) and Homology directed repair (HDR), which would produce three types of results as shown in Table 4. In the case of Number 3, it indicates that the nucleic acid molecule encoding the HLA-E restricted presenting peptide was successfully inserted into the B2M endogenous gene site between the nucleic acid encoding the B2M signal peptide and the B2M mature protein, and used the same open reading frame as the B2M endogenous gene. In this case, the cells would express the fusion protein of the HLA-E restricted presenting peptide and B2M instead of the wild-type B2M protein, resulting in that the presenting peptide binded in B2M and HLA heavy chain complex was no longer diverse, but fixed as HLA-E restricted presenting peptide. Therefore, there was only HLA-E complex on the cell surface, because HLA-ABC heavy chain could not bind to HLA-E restricted presenting peptide to form a complex. Therefore, the targeted insertion efficiency in the gene could be determined by detecting the expression of HLA-ABC and HLA-E in cells.

**Table 4. Categories of cell phenotypes after gene editing**

| Number | Type | HLA-A, HLA-B and HLA-C (*i.e.,* HLA-ABC) | HLA-E |
|---|---|---|---|
| 1 | Unsuccessfully edited | Positive | Positive |
| 2 | Successfully edited, repaired through NHEJ pathway, resulting in mismatch or frameshift (gene knock-out) | Negative | Negative |
| 3 | Successfully edited, repaired through HDR pathway, and the insertion was successful (HLA-E restricted presenting peptide gene was inserted) | Negative | Positive |

Culture was continued for 5 days after electrotransformation, that is, on the day 10 of the experiment, a small amount of cells was taken out, 1 mL of PBS (Gibco, cat# C10010500BT) was added to wash the cells once, then the cells were resuspended in 100 µL of PBS, and 3 µL of anti-HLA-A/B/C-APC (R&D, cat# FAB7098A) antibody and anti-HLA-E-PE (Biolegend, cat# 342604) antibody were added, the resulting mixture was incubated at 4°C for 30 minutes, 1 mL of PBS was added to wash the cells, centrifugation was performed at 300 g for 3 minutes, and the supernatant was removed. The cells were resuspended in 200 µL of PBS, washed and detected on a flow cytometer.

The detection results were shown in Fig. 2. The group without homologous recombination DNA produced gene knock-out efficiency, with HLA-ABC negative reaching 81.3% and no HLA-E expression. The group with homologous recombination DNA could detect HLA-ABC-negative and HLA-E-positive cell population. With the increase of DNA dosage, HLA-ABCHLA-E⁺ cells were 11.70%, 16.63% and 19.86% respectively. The results showed that after gene editing, the targeted insertion of HLA-E-restricted presenting peptide could be achieved through one-step electrotransformation, so that the cells could express HLA-E specifically while HLA-A, HLA-B and HLA-C were absent.

### Example 5 Verification of targeted insertion in primary T cells (DNA sequencing, gene-level detection)

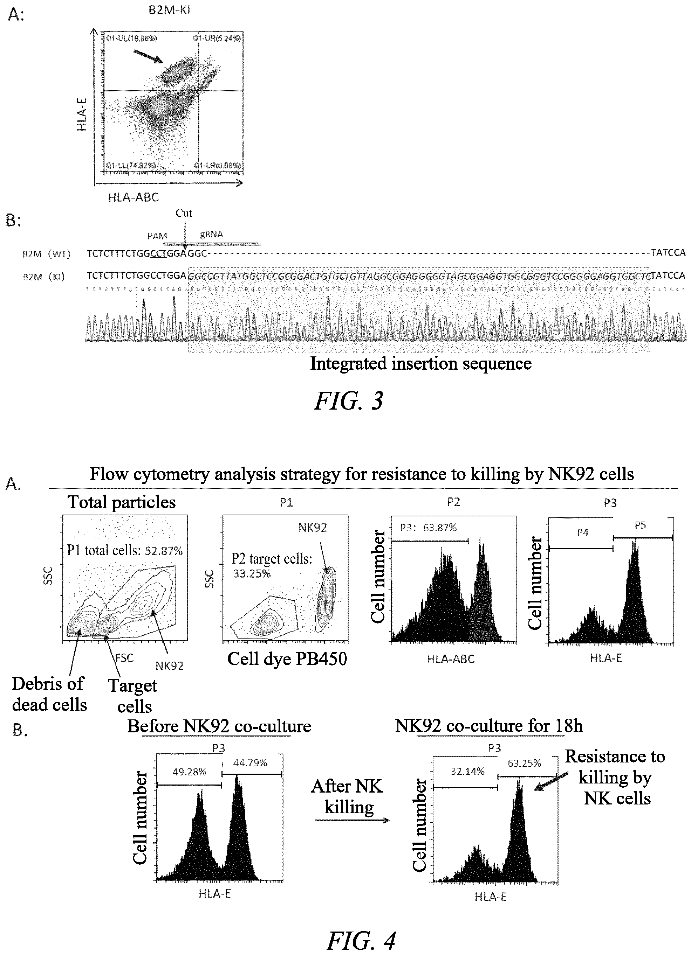

To further verify the targeted insertion of HLA-E-restricted presenting peptide, we performed DNA sequencing at the gene level. Considering that the efficiency of targeted insertion was between 10% and 20%, we enriched the target cells and performed DNA extraction and sequencing analysis on a purer cell subset. The cell subset indicated by the arrow in Fig. 3A, that is, the cell subset of HLA-ABC⁻HLA-E⁺, was sorted by flow cytometry.

The sequencing primer sequence was SEQ ID NO: 5: 5'AGCAAACTCACCCAGTCTAGT3'. The sequencing results were shown in Fig. 3B, showing that the target DNA sequence had been integrated into the *B2M* gene target site, and the open reading frame was correct.

### Example 6 Evaluation of the ability of T cells expressing HLA-E through gene knock-in to resist killing by NK cells

HLA-E transmits inhibitory signals to NK cells by interacting with NKG2A/CD94, thereby inhibiting the activation of NK cells and the killing of target cells. In order to functionally verify whether T cells that express HLA-E through gene knock-in have the ability to resist killing by NK cells, we used the method of co-culture of edited cells and NK92 cells for evaluation. NK92 is a model cell line derived from NK cells and is widely used in assays to evaluate NK cell-mediated killing.

The target cells to be killed were derived from the gene-edited T cells prepared in Example 3. As shown in Fig. 3A, the cells also comprise HLA-ABC⁻HLA-E⁺ cells (upper left, representing the knock-in subset) and HLA-ABC⁻HLA-E⁻ cells (lower left, representing the knock-out subset). Two types of cells were present at the same time. When cultured with NK92, changes in the proportion of the two types of cells would indicate the intensity of killing by NK92. The proportion of cells with weak NK92 killing would be significantly increased after co-culture.

### Flow cytometry detection method for killing by NK-92 cells:

The culture medium was ImmunoCultTM, and purchased from STEMCELL Company. The effector cells, NK-92 cells, were purchased from Shanghai Institute of Cell Research, Chinese Academy of Sciences. The target cells were derived from the gene-edited T cells prepared in Example 3. The final concentration of the target cells was 2 × 10⁴/ml. The NK92 cells were labeled with cell dye (PB450, Thermo Fisher Scientific, cat#C34557) before co-culture. NK-92 cells : target cells = 1 : 1 were mixed, and co-cultured in a 96-well culture plate at 100 µL per well in a 37°C incubator for 24 hours.

For flow cytometry detection, as shown in Fig. 4A, in the P1 gate, the cell dye PB450 could distinguish the NK92 cells and the target cells, and PB450-negative target cells P2 were selected for further analysis. Due to gene editing, the target cells would be divided into a HLA-ABC negative subset P3 and a positive subset. HLA-ABC negative represented cells that have been successfully edited (including knock-out and knock-in). Furthermore, in the P3 subset, the gene knock-in subset P5 and the subset P4 that was only edited without knock-in could be distinguished by the expression of HLA-E. Comparing the changes in P4 and P5 cell subsets after killing by NK92 cells could determine the killing status.

As shown in Fig. 4B, the proportions of HLA-ABC⁻HLA-E⁻ subset P4 and HLA-ABC⁻HLA-E⁺ subset P5 were 49.28% and 44.79% respectively, which changed to 32.14% and 63.25% after co-culture and killing. In the same co-culture conditions, the proportion of HLA-E⁺ cells increased significantly, suggesting that this cell subset had a stronger ability to resist killing by NK92 cells.

Further, we sorted and purified HLA-ABC⁻HLA-E⁻ and HLA-ABC⁻HLA-E⁺ cells which were co-cultured with NK92 cells respectively, the killing rate was calculated, and the activity of the gene knock-in cell subset in resisting killing by NK cells was quantitatively determined. The cells were derived from the gene-edited cells prepared in Example 3, in which HLA-ABC⁻HLA-E⁻ were derived from the group numbered 2 in Table 3, which was recorded as the RNP group. HLA-ABC⁻HLA-E⁺ were derived from the group numbered 5 in Table 3 (corresponding to the cells indicated by the arrows in Fig. 3A), which was recorded as the RNP-KI (Knock In) group. The co-culture method was the same as the above method in this example, and the groups were as shown in Table 5:

**Table 5. NK92 killing test design**

| Group | Effector cell to target cell ratio | (RNP) HLA-ABC-HLA-E- | | | (RNP-KI) HLA-ABC-HLA-E+ | | |
|---|---|---|---|---|---|---|---|
| Target cells alone culture group | 0 : 1 | Repeat 1 | Repeat 2 | Repeat 3 | Repeat 1 | Repeat 2 | Repeat 3 |
| NK92 co-culture group | 0.1 : 1 | Repeat 1 | Repeat 2 | Repeat 3 | Repeat 1 | Repeat 2 | Repeat 3 |
| | 0.5 : 1 | Repeat 1 | Repeat 2 | Repeat 3 | Repeat 1 | Repeat 2 | Repeat 3 |
| | 1 : 1 | Repeat 1 | Repeat 2 | Repeat 3 | Repeat 1 | Repeat 2 | Repeat 3 |
| | 2: 2 | Repeat 1 | Repeat 2 | Repeat 3 | Repeat 1 | Repeat 2 | Repeat 3 |

Killing rate (%) = (1 -average of NK92 co-culture group/average of target cells alone culture group) × 100% (The statistical method used T-test, two-tailed, equal sample variance assumption method)

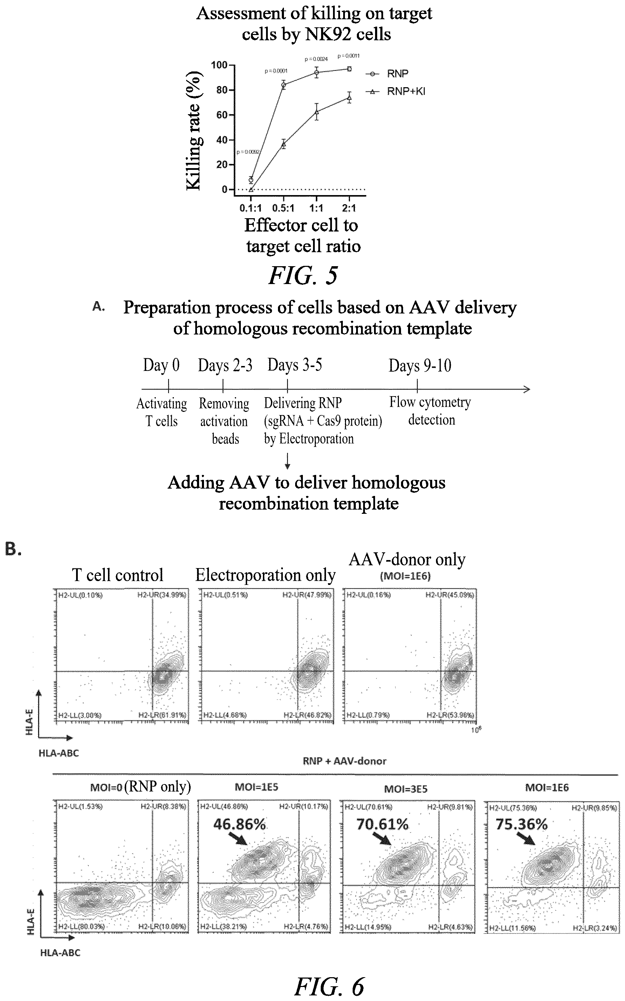

The results were shown in Fig. 5. Under the conditions of each effector cell to target cell ratio, the resistance activity of the gene-inserted subset (HLA-ABC⁻HLA-E⁺) against NK was significantly higher than that of the subset without expressing HLA-E (HLA-ABC⁻HLA-E⁻).

### Example 7 Establishment of a method for gene knock-in using AAV to deliver homologous recombination templates

In this example, adeno-associated virus (AAV) is used to deliver a single-stranded DNA as a homologous recombination template to perform gene knock-in.

### AAV virus preparation:

(1) The packaging plasmid comprised the AAV core plasmid pAAV, and the backbone was derived from the plasmid Addgene ID: #51905, the packaging helper plasmid pRepCap6 was derived from plasmid Addgene ID: #110770 and the pHelper was derived from plasmid Addgene ID: #112867, AAV serotype AAV6 was selected, and virus packaging was performed in HEK293T cells (purchased from Shanghai Institute of Cell Research, Chinese Academy of Sciences). The exogenous homologous recombination DNA sequence was SEQ ID NO: 6. After the whole gene was synthesized, it was digested with NdeI/HpaI double enzyme and ligated into the pAAV vector for subsequent AAV packaging preparation.
(2) HEK293T preparation

The HEK 293T cells were splited one day in advance, and when packaging, the cell density was 85%-90% and the cells were evenly distributed and in good condition (that is, under the microscope, there were no impurities, no cells suspended or a few cells suspended in the culture medium, and the cells were full and adhered evenly in the culture dish).

Packaging: the cell medium was replaced one to two hours in advance with serum-free DMEM (Merck, cat#D5546) medium (1% HEPES and 1% P/S).

Transfection: taking the amount of five 15 cm culture dishes as an example, to the 15 ml centrifuge tube was added in sequence DMEM: 9 ml, pHelper: 124 µg, pRepCap6: 76 µg, pAAV: 65.1 µg, PEI: 1 ml, and then the liquid was shaken and mixed well and let stand at room temperature for 30 minutes. The above liquid after standing was added evenly to 5 dishes of HEK293T cells and the dishes were labeled. The liquid was added slowly against the wall to avoid floating the cells. After adding, the resulting liquid was shaken and mixed well and placed in an incubator at 37°C, 5% CO₂ for culture.

Collection: After 72 hours, the cells were floated and collected together with the culture medium into a 50 ml centrifuge tube. Cells were collected into three 50 ml centrifuge tubes every 5 dishes, with an average of 45 ml per tube, and centrifugation was performed at 3500 rpm (2100 g) at room temperature for 7 min. The culture supernatant was transferred to a new tube, and precipitated with PEG8000 (Merck, cat#P2139-500G) overnight (adding 2.33 g NaCl + 8.5 g PEG8000 per 100 mL). The next day, centrifugation was performed at 3500 g and 4°C for 30 min. The supernatant was discarded and the precipitate was resuspended in 2 ml of PBS + 0.001% PF68. The cell precipitate was resuspended in 5 ml of PBS + 0.001% PF68, frozen and thawed once, then 2 ml of 5 M Nacl (autoclave) was added, and vortexed to mix. The resuspension of B and the resuspension of C was mixed, shaken and mixed well, and then sonicated until the mixture was no longer viscous after repeated sonication. The sonicated liquid was centrifuged at 3500 g and 4°C for 30 min, and the supernatant was transferred to a new centrifuge tube.

Ultracentrifugation purification: iodixanol density gradient was used for centrifugation purification. Different concentrations of iodixanol (CAS:92339-11-2) were formulated according to the information in Table 6. 60% iodixanol w/v was formulated with sterile water and filtered into a sterilized bottle using a 0.2 µm filter membrane.

**Table 6. Iodixanol solution formulation ratio**

| Number of layers | 60% iodixanol (ml) | 5 M NaCl (ml) | H₂O (ml) | 2.5 M KCl (µl) | 1 M MgCl₂(µl) | 10*PBS (ml) | Total volume (ml) |
|---|---|---|---|---|---|---|---|
| 15% | 25 | 20 | 45 | 100 | 100 | 10 | 100 |
| 25% | 41.7 | 0 | 48.3 | 100 | 100 | 10 | 100 |
| 40% | 66.7 | 0 | 23.3 | 100 | 100 | 10 | 100 |
| 60% | 100 | 0 | 0 | 100 | 100 | 0 | 100 |

An ultracentrifuge tube (the ultracentrifuge tube and lid were autoclaved before use) was taken, and different concentrations of iodixanol were added layer by layer. Firstly 4.2 ml of the 60% layer was added, then 5 ml of the 40% layer was added, then 6 ml of the 25% layer was added, and finally 9 ml of the 15% layer was added. When adding different concentrations of iodixanol, the centrifuge tube was tilted and the iodixanol was injected slowly to avoid layer crossing. The entire operation needed to be performed in a biological safety cabinet. The processed crude virus solution was added to the upper layer. When adding the virus, especially at the beginning, the processed crude virus solution should be added carefully and slowly, and the tube should be covered tightly. Ultracentrifugation was performed at 48,000 rpm and 4°C for 2.5 h. The concentrated AAV virus was resuspended in 100 µl of PBS and placed in a 4°C refrigerator.

### Gene knock-in based on AAV delivery of homologous recombination template in primary T cells:

The process was shown in Fig. 6A. Primary T cells were derived from peripheral blood mononuclear cells (PBMCs) of healthy volunteers. The culture medium used was complete culture medium, ImmunoCult^{™}-XF T Cell Expansion Medium (Stem Cell Technology, cat#10981) + 300 IU/ml IL2 (Cayan, cat# HEILP-0201c). The T cells were activated using Dynabeads (Thermo, cat#11141D), Dynabeads : cells = 3 : 1. 24 h after activation, the T cells exhibited obviously clustering and enlargement in morphology. 48 h after T cell activation, 3E5 cells were taken and placed in a 24-well culture plate, and the activation magnetic beads were removed using a magnetic stand. RNP electrotransformation was performed on day 4 after activation. The sgRNA/Cas9 and the electrotransformation method were the same as in Example 3. 2 hours after electrotransformation, different doses of AAV were added to deliver homologous recombination templates. MOI (Multiplicity of Infection, ratio of virus amount to cell number) are 0, 1E5, 3E5, and 1E6 in order. On day 9 of the cell culture, detection of gene editing and exogenous gene insertion was performed. A small amount of cells was taken, 1 mL of PBS (Gibco, cat# C10010500BT) was added to wash the cells once, then the cells were resuspended in 100 µL of PBS; 1 mL of PBS (Gibco, cat# C10010500BT) was added to wash the cells once, then the cells were resuspended in 100 µL of PBS; 3 µL of anti-HLA-ABC-APC (R&D, cat# FAB7098A) antibody and anti-HLA-E-PE (Biolegend, cat#342604) antibody were added, the resulting mixture was incubated at 4°C for 30 minutes, 1 mL of PBS was added to wash the cells, centrifugation was performed at 300 g for 3 minutes, and the supernatant was removed. The cells were resuspended in 200 µL of PBS, washed and detected on a flow cytometer.

The test results were shown in Fig. 6B. In the T cell control group, the electroporation only group, and the group of delivery of the homologous recombination template using only the AAV-donor, no gene knock-out or site-targeted insertion was detected. The group without homologous recombination template at MOI = 0 produced gene knock-out, with HLA-ABC negative reaching 80.03% and no HLA-E expression. The group with homologous recombination AAV template could detect HLA-ABC negative and HLA-E positive cell population. As shown by the arrow, with the increase of MOI, HLA-ABC⁻ HLA-E⁺ cells were 46.86%, 70.61% and 75.36% respectively. The results showed that using AAV as a homologous recombination template, after gene editing, the targeted insertion of HLA-E-restricted presenting peptide could be achieved, so that the cells could express HLA-E specifically while the expression of HLA-A, HLA-B and HLA-C was absent, and there was a dose effect, and the efficiency was higher than the method using DNA as a template (Example 4).

### Example 8 Preparation and detection of universal CAR-T (UCAR-T) cells based on AAV delivery

This example utilized the established CRISPR/Cas9-AAV delivery method to simultaneously achieve TCR/CD3 complex knock-out, HLA-ABC knock-out, and HLA-E overexcession in CAR-T cells by gene-targeted insertion through one-step gene editing.

**CRISPR/Cas9 system:** The sources were the same as those in Example 3. That is, the Cas9 protein was derived from wild type of *Streptococcus pyogenes* (SpCas9), had a nuclear localization signal (NLS), and was purchased from Integrated DNA Technologies (catalog number: 1074181). The sequence of the gRNA targeting TRAC was shown in SEQ ID NO: 7, and the sequence of the gRNA targeting B2M was the same as that in Example 3 and shown in SEQ ID NO: 3. The AAV used to deliver the homologous recombination template was the same as that in Example 7, and the nucleotide sequence was shown in SEQ ID NO: 6.

Lentiviral packaging system for CAR delivery: A three-plasmid system comprising Lentiviral expression plasmid of interest (Addgene ID: #12252), packaging helper plasmids psPAX2 (Addgene ID: #12260) and pMD2.G (Addgene ID: #12259). The constructed CAR sequence targeting CD19 was named CJP, and the sequence was shown in SEQ ID NO: 8. After the whole CJP gene was synthesized, it was digested with BamHI/SalI and ligated into the viral expression plasmid (Addgene ID: #12252).

Virus packaging was performed in HEK293T cells (purchased from Shanghai Institute of Biochemistry and Cell Biology, Chinese Academy of Sciences). The preparation process was as follows: HEK293T cells were resuscitated from cryopreserved working cells and cultured in a 10 cm culture dish with a DMEM medium (+10% FBS+1% P/S) (Cellgro 10-013-CMR). The medium was changed after 2 days of resuscitation. After the cells grew to fill the culture dish, passage was performed (generally, cells growing to fill one culture dish may be transferred to five culture dishes), and plasmid transfection can be performed after 4 passages. According to the preferred transfection of the system, PEI as a transfection reagent and a condition of PEI: plasmid (mass ratio) = 2 : 1 were used for transfection. A mixture of the plasmid and PEI was added to an Opti-MEM medium (Gibco, cat#31985-070), and then the mixture solution was added to the 4th generation HEK293T cells after passages. 6 h after transfection, the medium was replaced with a fresh medium containing 2% FBS, the cells were then cultured for another 72 h, and the supernatant of the HEK293T cells was collected. The collected viral supernatant was concentrated by ultracentrifugation (82200 g, centrifuging at 4°C-8°C for 2 h), and the concentrated virus was filtered with a 0.22 µm filter membrane and sterilized, and then resuspended for later use.

### Preparation of UCAR-T cells based on AAV delivery of homologous recombination template:

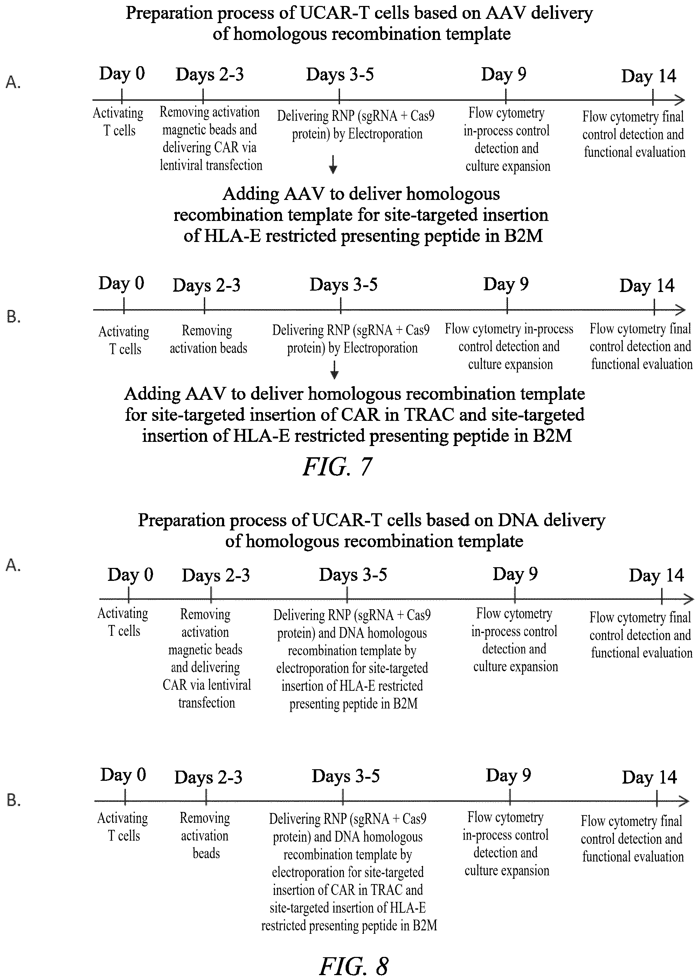

The cell preparation process was shown in Fig. 7. In Fig. 7, Fig. 7A showed that CAR was delivered by lentivirus at 48 h after T cell activation; HLA-E was delivered 3-5 days after T cell activation by CRIPSR/Cas9-AAV targeted insertion method, and TCR/CD3 was knocked out at the same time. Another strategy, as shown in Fig. 7B, the CRIPSR/Cas9-AAV targeted insertion method was adopted for both CAR and HLA-E, that is, targeted insertion of CAR into the TRAC gene locus, and targeted insertion of HLA-E into the B2M gene locus. Simultaneous knock-out of TCR/CD3 and integrated expression of dual-site genes was achieved through one-step electrotransformation.

Primary T cells were derived from peripheral blood mononuclear cells (PBMCs) of healthy volunteers. The culture medium used was complete culture medium, ImmunoCult^{™}-XF T Cell Expansion Medium (Stem Cell Technology, cat#10981) + 300 IU/ml IL2 (Cayan, cat# HEILP-0201c). T cells were activated by Dynabeads (Thermo, cat#11141D), Dynabeads : cells = 3 : 1. 24 h after activation, the T cells exhibited obviously clustering and enlargement in morphology. 48 hours after T cell activation, a magnetic stand was used to remove the activation magnetic beads, the 3E5 cells were taken and cultured in a 24-well culture plate; the prepared lentivirus was added in an amount 3 times the number of the cells, *i.e.,* MOI=3; and the medium was supplemented to 500 µL. After 24 h, the medium was supplemented to 1 mL, which was beneficial to cell growth. RNP electrotransformation was performed on day 4 after activation. The sgRNA/Cas9 and the electrotransformation method were similar to those in Example 3. In this example, in addition to adding the sgRNA targeting B2M, an equal dose of sgRNA targeting TRAC needed to be added. 2 hours after electrotransformation, different doses of AAV were added to deliver homologous recombination templates. The MOI was 0, 1E5, 3E5, and 1E6 in sequence for culture. In this example, 1E6 was preferred.

On day 9 of the cell culture, detection of gene editing and exogenous gene insertion was performed. A small amount of cells was taken, 1 mL of PBS (Gibco, cat# C10010500BT) was added to wash the cells once, then the cells were resuspended in 100 µL of PBS; 1 mL of PBS (Gibco, cat# C10010500BT) was added to wash the cells once, then the cells were resuspended in 100 µL of PBS; 3 µL of anti-FMC63 primary antibody (to detect CAR19) was added, the resulting mixture was incubated at 4°C for 30 minutes, 1 mL of PBS was added to wash the cells, centrifugation was performed at 300 g for 3 minutes, and the supernatant was removed. The cells were resuspended in 100 µL of PBS, added with 0.5 µL of a second antibody for detecting CAR19, 3 µL of an anti-HLA-ABC antibody, 3 µL of an anti-CD3 antibody (for detecting gene editing efficiency) and 3 µL of an anti-HLA-E antibody, uniformly mixed, and incubated at 4°C for 30 min. After washing, the cells were detected by flow cytometry.

UCAR-T cells could also be prepared based on DNA delivery of homologous recombination templates. The preparation process was shown in Fig. 8.

### Example 9 Comparison of preparation of UCAR-T cells by different methods

For UCAR-T cells for gene knockout, the preparation method by conventional lentiviral transfection was used: referring to Example 7.3 of patent WO 2021/027758 A1. UCAR-T that only expressed CAR was designated as preparation method 1 (UCART), UCAR-T that co-expressed CAR and rejection-resistant chimeric CM (HLA-E restricted presentating peptide-B2M) was designated as preparation method 2 (UCART-CM).

The preparation method was briefly described as follows. Primary T cells were derived from peripheral blood mononuclear cells (PBMCs) of healthy volunteers. T cells were activated by Dynabeads, Dynabeads : cells = 3 : 1. 48 hours after T cell activation, a magnetic stand was used to remove the activation magnetic beads, the 3E5 cells were taken and cultured in a 24-well culture plate; the prepared lentivirus was added in an amount 3 times the number of the cells, *i.e.,* MOI = 3. Lentivirus delivered CAR19 or CAR19 co-expressed with CM. After adding lentivirus, the culture medium was supplemented to 500 µL. After 24 h, the medium was supplemented to 1 mL, which was beneficial to cell growth. On day 4 after activation, RNP electrotransformation was performed, and sgRNAs targeting TRAC and B2M were used to simultaneously knock out the TCR/CD3 complex and the HLA-ABCB2M complex. UCAR-T cells that expressed CAR or expressed CAR19/HLA-E and were TCR and HLA-ABC double negative were finally generated.

At the same time, the method of Example 8 of this patent was used to prepare universal CAR-T cells (recorded as AAV site-targeted integration: UCART-KI) based on CRISPR/Cas9 targeted insertion method. At the same time, a "control without gene editing: CAR-T" control group of cells was prepared, and T cells were only subjected to lentiviral transfection of CAR without the gene editing step. Each group was compared head-to-head to evaluate the CAR and HLA-E expression efficiency of UCAR-T cells prepared by different methods.

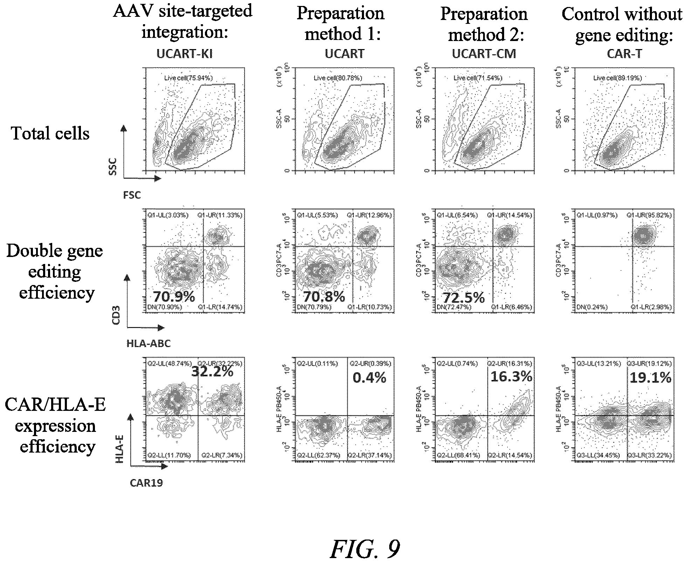

The results of cell phenotype testing (day 14) before functional evaluation were shown in Fig. 9. Among different prepared UCAR-T, the proportion of CD3/HLA-ABC double gene knock-out was similar, that is, for method 1, it was 70.8%, and for method 2, it was 72.5%, and for the AAV targeted integration method of the present invention, it was 70.9% (second row); Regarding the comparison of the co-expression efficiency of CAR and HLA-E, for the cells prepared by the AAV targeted integration method of the present invention, it was 32.2%, which was significantly higher than that of other groups - for method 1, it was 0.4%, and for method 2, it was 16.3%. It was worth noting that the AAV targeted integration method of the present invention was significantly stronger in terms of the abundance of HLA-E expression, and the flow cytometry detection showed obvious clustering, as indicated by the arrow in the upper right cluster (third row). The above results showed that the cells prepared by the AAV targeted integration method of the present invention had higher expression efficiency and greater abundance of CAR and HLA-E, suggesting that they had stronger resistance to rejection.

On day 14 of the culture of the UCART obtained in this example, the functional evaluation of Examples 10 and 11 was also performed.

### Example 10 Detection of the ability to resist allogeneic PBMC killing

In this example, the proliferation ability of edited UCAR-T cells in an allogeneic PBMC environment was detected, and in the environment, allogeneic immunological rejection (not only from NK cells) was present, and target cells (tumor Raji cells) were also present.

UCAR-T cells were derived from each group of cells prepared in Example 9. The cells were co-cultured, UCAR-T cells : tumor Raji cells : allogeneic PBMC = 1 : 5 : 20. The number of CAR positive cells in UCAR-T cells was 5E4 cell/ml, and other cells were extrapolated proportionally. The cells were cultured in a 24-well culture plate at 37°C. The number of CAR positive cells in different groups was recorded at different time points. Before co-culture of allogeneic PBMC and Raji cells, staining was performed with Dye eFluorTM 670 to distinguish universal CAR-T cells: the cell density was adjusted to 1E7 cells/ml. e670 dye with a final concentration of 10 µM was added, and the cells were incubated at room temperature in the dark for 5 min, washed with a medium three times, and then used in experiments. The cells were sampled on days 0, 3, 5, 7, and 9 of co-culture, and detected by flow cytometry, and the number of CAR positive cells proliferated or remained was counted.

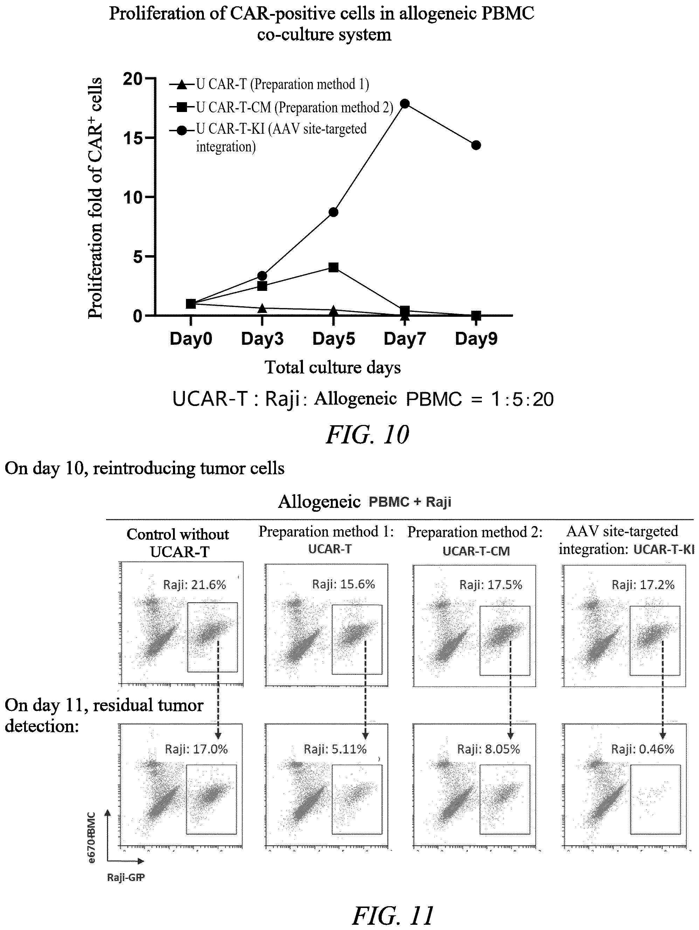

As shown in Fig. 10, the UCAR-T cells prepared by method 1 cannot be proliferated in the allogeneic PBMC co-culture system because they were HLA-ABC negative and had no HLA-E expression (Fig. 9), and the number of cells gradually decreased, suggesting that they were strongly rejected by allogeneic PBMC; the UCAR-T cells prepared by method 2 expressed a certain proportion of HLA-E and had the ability to proliferate in the first 5 days, and then the number of cells gradually decreased, suggesting that they had a certain resistance to rejection activity, but the effect was limited; the UCAR-T cells prepared by the AAV targeted integration of the present invention showed stronger proliferation ability under the same conditions, and still maintained an proliferation number of more than 10 times on day 9, suggesting that the cells prepared by this method had stronger rejection resistance ability and proliferation ability.

### Example 11 Detection of tumor cell killing activity

This example is a continuation of Example 10. As shown in Fig. 11, on day 10 of allogeneic PBMC co-culture, 2E5 Raji tumor cells were introduced to simulate tumor recurrence, and the killing ability of each group against tumor cells was tested. Under the condition of similar initial tumor cells, after 24 hours of killing, the UCART-KI group prepared by AAV targeted integration method showed stronger killing activity on tumors, the proportion of the tumor cells was decreased from the initial 17.2% to 0.64%, while the UCART and UCART-CM groups prepared by methods 1 and 2 had limited killing effect on tumor cells, the proportion of the tumor cells was decreased from 15.6% to 5.11% and 17.5 to 8.05% respectively, and the control group without UCAR-T had the weakest killing effect, the proportion of the tumor cells was decreased from 21.6% to 17.0%. The above data reflected that the UCART-KI group prepared by the AAV targeted integration method still had effective sustained killing ability after 10 days of allogeneic PBMC killing, could kill tumor cells efficiently, and had obvious advantages in anti-rejection and tumor killing activities.

The foregoing detailed description is provided by way of explanation and example, and is not intended to limit the scope of the appended claims. At present, the various changes in the examples listed in the present application would have been obvious to an ordinary person skilled in the art, and are within the scope of the appended claims and the equivalents thereof.

## Claims

1. A modified cell comprising a polynucleotide that encodes a presenting peptide, wherein the polynucleotide is under the transcriptional regulation of an endogenous B2M gene.

2. The modified cell according to claim 1, wherein the polynucleotide is in the locus of the endogenous B2M gene.

3. The modified cell according to any one of claims 1-2, wherein the modified cell is capable of expressing a B2M protein.

4. The modified cell according to claim 3, wherein the presenting peptide is capable of covalently binding to the B2M protein.

5. The modified cell according to any one of claims 1-4, wherein the modified cell is capable of expressing a B2M protein, and at least a portion of the B2M protein is encoded by the endogenous B2M gene.

6. The modified cell according to any one of claims 1-5, wherein the modified cell is capable of expressing a B2M protein, and the B2M protein is substantially encoded by the endogenous B2M gene.

7. The modified cell according to any one of claims 3-6, wherein the expression and/or activity of the B2M protein on the cell surface is substantially not down-regulated compared with cells without the modification.

8. The modified cell according to any one of claims 3-7, wherein the expression and/or activity of the B2M protein on the cell surface is down-regulated by no more than about 80%, about 70%, about 60%, about 50%, about 40%, about 30%, about 20% or less compared with cells without the modification.

9. The modified cell according to any one of claims 3-8, wherein the polynucleotide further comprises a nucleic acid molecule encoding a fragment of the B2M protein.

10. The modified cell according to any one of claims 1-9, wherein the polynucleotide does not comprise a complete B2M gene coding region.

11. The modified cell according to any one of claims 1-10, wherein the polynucleotide does not comprise a nucleic acid molecule encoding a heavy chain of an MHC molecule.

12. The modified cell according to any one of claims 1-11, wherein the polynucleotide is in the same open reading frame as the endogenous B2M gene.

13. The modified cell according to any one of claims 1-12, wherein the polynucleotide is operably linked to a promoter of the endogenous B2M gene.

14. The modified cell according to any one of claims 1-13, wherein the polynucleotide is located between a nucleic acid molecule encoding a signal peptide and a nucleic acid molecule encoding a B2M mature protein.

15. The modified cell according to any one of claims 1-14, wherein the modified cells have reduced allogeneic rejection response compared to cells without the modification.

16. The modified cell according to any one of claims 3-15, wherein the presenting peptide is capable of forming a complex with the B2M protein and the heavy chain of the MHC molecule.

17. The modified cell according to claim 16, wherein the complex is capable of inhibiting immune cell activation.

18. The modified cell according to claim 17, wherein the immune cell is selected from the group comprising a natural killer (NK) cell, a T lymphocyte, a B lymphocyte, a NKT cell, a monocyte, a macrophage, a mast cell, a granulocyte, a dendritic cell, a lymphocyte, a leukocyte and/or a peripheral blood mononuclear cell.

19. The modified cell according to any one of claims 17-18, wherein the immune cell is a natural killer (NK) cell, a NKT cell and/or a T lymphocyte.

20. The modified cell according to any one of claims 17-19, wherein the inhibiting immune cell activation comprises inhibiting an immune cell from participating in an immune response.

21. The modified cell according to any one of claims 17-20, wherein the inhibiting immune cell activation comprises binding to an inhibitory receptor of the immune cell.

22. The modified cell according to claim 21, wherein the inhibitory receptor of the immune cell is selected from the group comprising NKG2A, NKG2B, KIR2DL1, KIR2DL2, KIR2DL3, KIR2DL4, KIR2DL5, KIR3DL1, KIR3DL2 and KIR3DL3.

23. The modified cell according to any one of claims 11-22, wherein the heavy chain of the MHC molecule comprises a heavy chain of a non-classical MHC class I molecule and/or a heavy chain of an MHC-like molecule.

24. The modified cell according to claim 23, wherein the heavy chain of the non-classical MHC class I molecule comprises an HLA-E heavy chain, an HLA-F heavy chain and/or an HLA-G heavy chain.

25. The modified cell according to any one of claims 23-24, wherein the heavy chain of the MHC-like molecule comprises a CD1 heavy chain, an MR1 heavy chain, an FcRn heavy chain and/or a UL18 heavy chain.

26. The modified cell according to any one of claims 23-25, wherein the heavy chain of the MHC molecule is an HLA-E heavy chain.

27. The modified cell according to any one of claims 16-26, wherein the presenting peptide is capable of binding to the peptide binding groove of the heavy chain of the MHC molecule.

28. The modified cell according to any one of claims 1-27, wherein the presenting peptide is derived from a virus, a prokaryote, an eukaryote or a mammal.

29. The modified cell according to any one of claims 1-28, wherein the presenting peptide is derived from a human.

30. The modified cell according to any one of claims 1-29, wherein the presenting peptide comprises a non-classical MHC class I restricted presenting peptide and/or an MHC-like molecule restricted presenting peptide.

31. The modified cell according to any one of claims 1-30, wherein the presenting peptide is a non-classical MHC class I restricted presenting peptide.

32. The modified cell according to any one of claims 1-30, wherein the presenting peptide is an HLA-E restricted presenting peptide.

33. The modified cell according to any one of claims 1-32, wherein the presenting peptide is derived from a protein selected from the group comprising an ATP-binding cassette transporter, a multidrug resistance-associated protein 7, gliadin, an HSP60 conserved region and a GroEL conserved region.

34. The modified cell according to any one of claims 1-33, wherein the presenting peptide is derived from a signal peptide of a MHC class I molecule, or a fragment thereof.

35. The modified cell according to any one of claims 1-34, wherein the presenting peptide is derived from a signal peptide of a protein selected from the group comprising HLA-Al, HLA-A2, HLA-A*02, HLA-A*0203, HLA-A*0210, HLA-A3, HLA-A*23, HLA-A*24, HLA-A*2403, HLA-A*2410, HLA-A*2502, HLA-A*2501, HLA-A*26, HLA-A*66(01,02), HLA-A9, HLA-A*6603, HLA-A10, HLA-A*11, HLA-A19, HLA-A*29, HLA-A*30, HLA-A*31, HLA-A*32, HLA-A*33, HLA-A*74, HLA-A*7403, HLA-A28, HLA-A36, HLA-A34, HLA-A43, HLA-A*3401, HLA-A*80, HLA-B5, HLA-B7, HLA-B8, HLA-B12, HLA-B13, HLA-B14, HLA-B15, HLA-B16, HLA-B17, HLA-B18, HLA-B21, HLA-B22, HLA-B27, HLA-B35, HLA-B37, HLA-B38, HLA-B39, HLA-B40, HLA-B41, HLA-B42, HLA-B46, HLA-B47, HLA-B48, HLA-B49, HLA-B50, HLA-B51, HLA-B52, HLA-B53, HLA-B54, HLA-B55, HLA-B56, HLA-B57, HLA-B58, HLA-B59, HLA-B60, HLA-B61, HLA-B62, HLA-B63, HLA-B64, HLA-B65, HLA-B67, HLA-B70, HLA-B71, HLA-B73, HLA-B75, HLA-B76, HLA-B77, HLA-B78, HLA-B81, HLA-B82, HLA-B83, HLA-Cw1, HLA-Cw2, HLA-Cw3, HLA-Cw4, HLA-Cw5, HLA-Cw6, HLA-Cw7, HLA-Cw8, HLA-Cw9, HLA-Cw10, HLA-Cw*0809, HLA-Cw7, HLA-Cw*1701, HLA-G and HLA-F.

36. The modified cell according to any one of claims 1-35, wherein the presenting peptide is 5-30 amino acids in length.

37. The modified cell according to any one of claims 1-36, wherein the presenting peptide is 7-20 amino acids in length.

38. The modified cell according to any one of claims 1-37, wherein the presenting peptide is 8-10 amino acids in length.

39. The modified cell according to any one of claims 1-38, wherein the presenting peptide comprises an amino acid sequence as shown in SEQ ID NO: 17.

40. The modified cell according to any one of claims 1-39, wherein the presenting peptide comprises an amino acid sequence as shown in any one of SEQ ID NOs: 18-70.

41. The modified cell according to any one of claims 1-40, wherein the presenting peptide comprises an amino acid sequence as shown in SEQ ID NO: 35, SEQ ID NO: 30 or SEQ ID NO: 33.

42. The modified cell according to any one of claims 1-41, wherein the polynucleotide further comprises a nucleic acid molecule encoding a linker.

43. The modified cell according to claim 42, wherein in an expression product of the modified cell, the presenting peptide and the B2M protein are linked via the linker.

44. The modified cell according to any one of claims 42-43, wherein the linker comprises a rigid linker and/or a flexible linker.

45. The modified cell according to any one of claims 42-44, wherein the linker comprises an amino acid sequence as shown below: (EAAAK)ₙ, where n is 3, 4, or 5.

46. The modified cell according to any one of claims 42-45, wherein the linker comprises an amino acid sequence as shown below: (GGGGS)ₙ, where n is 3, 4, or 5.

47. The modified cell according to any one of claims 42-46, wherein the linker comprises an amino acid sequence as shown in any one of SEQ ID NOs: 71-99.

48. The modified cell according to any one of claims 42-46, wherein the linker comprises an amino acid sequence as shown in SEQ ID NO: 99.

49. The modified cell according to any one of claims 42-47, wherein the linker is located at the C-end of the presenting peptide.

50. The modified cell according to any one of claims 1-49, wherein the polynucleotide does not comprise a nucleic acid molecule encoding a B2M protein or a fragment thereof.

51. The modified cell according to any one of claims 1-50, wherein the polynucleotide encodes an amino acid sequence as shown in SEQ ID NO: 9.

52. The modified cell according to any one of claims 1-51, wherein the polynucleotide comprises a nucleotide sequence as shown in SEQ ID NO: 12.

53. The modified cell according to any one of claims 1-52 for use in cell transplantation, tissue transplantation and/or organ transplantation.

54. The modified cell according to any one of claims 1-53, wherein the modified cell comprises a mammalian cell.

55. The modified cell according to any one of claims 1-54, wherein the modified cell comprises a stem cell and/or a somatic cell.

56. The modified cell according to any one of claims 1-55, wherein the modified cell comprises a blood cell, a muscle cell, a nerve cell, and/or an immune cell.

57. The modified cell according to any one of claims 1-56, wherein the modified cell comprises an immune cell selected from the group comprising a natural killer (NK) cell, a T lymphocyte, a B lymphocyte, a NKT cell, a monocyte, a macrophage, a mast cell, a granulocyte, a dendritic cell, a lymphocyte, a leukocyte and/or a peripheral blood mononuclear cell.

58. The modified cell according to any one of claims 1-57, wherein the modified cell is a natural killer (NK) cell, an NKT cell and/or a T lymphocyte.

59. The modified cell according to any one of claims 1-58, wherein the expression of a classical MHC class I molecule on the cell surface of the modified cell is down-regulated, and the expression of a non-classical MHC class I molecule is positive.

60. The modified cell according to any one of claims 1-59, wherein the expression of a classical MHC class I molecule on the cell surface of the modified cell is down-regulated, and the expression of a non-classical MHC class I molecule is substantially not down-regulated.

61. The modified cell according to any one of claims 1-60, wherein the expression of a classical MHC class I molecule on the cell surface of the modified cell is down-regulated, and the expression of a non-classical MHC class I molecule is up-regulated.

62. The modified cell according to any one of claims 1-61, wherein the expression and/or activity of a T cell receptor α constant region protein (TRAC) of the modified cell is down-regulated.

63. The modified cell according to any one of claims 1-62, wherein the modified cell further comprises a chimeric antigen receptor (CAR), a T cell receptor (TCR), a chimeric autoantibody receptor (CAAR), a TCR receptor fusion construct (TRuC), a T cell antigen coupler (TAC), an antibody TCR receptor (AbTCR), a chimeric CD3ε receptor and/or at least one synthetic receptor.

64. A method for preparing a modified cell, wherein the method comprises:
inserting a polynucleotide encoding a presenting peptide into the cell such that the polynucleotide is under the transcriptional regulation of an endogenous B2M gene.

65. The method according to claim 64, wherein the modified cell is capable of expressing a B2M protein.

66. The method according to any one of claims 64-65, wherein the presenting peptide is capable of covalently binding to the B2M protein.

67. The method according to any one of claims 64-66, wherein the modified cell is capable of expressing the B2M protein, and at least a portion of the B2M protein is encoded by the endogenous B2M gene.

68. The method according to any one of claims 64-67, wherein the modified cell is capable of expressing the B2M protein, and the B2M protein is substantially encoded by the endogenous B2M gene.

69. The method according to any one of claims 65-68, wherein the inserting does not substantially down-regulate the expression and/or activity of the B2M protein on the surface of the modified cell compared with cells without the modification.

70. The method according to any one of claims 65-69, wherein the inserting reduces the expression and/or activity of the B2M protein on the surface of the modified cell by no more than 80%, about 70%, about 60%, about 50%, about 40%, about 30%, about 20% or less compared with cells without the modification.

71. The method according to any one of claims 65-70, wherein the polynucleotide further comprises a nucleic acid molecule encoding a fragment of the B2M protein.

72. The method according to any one of claims 64-71, wherein the polynucleotide does not comprise a complete B2M gene coding region.

73. The method according to any one of claims 64-72, wherein the polynucleotide does not comprise a nucleic acid molecule encoding a heavy chain of an MHC molecule.

74. The method according to any one of claims 64-73, wherein the inserting comprises inserting the polynucleotide into the locus of the endogenous B2M gene.

75. The method according to any one of claims 64-74, wherein the inserting comprises inserting the polynucleotide into the same open reading frame as the endogenous B2M gene.

76. The method according to any one of claims 64-75, wherein the inserting operably links the polynucleotide to a promoter of the endogenous B2M gene.

77. The method according to any one of claims 64-76, wherein the inserting comprises inserting the polynucleotide between a nucleic acid molecule encoding a signal peptide and a nucleic acid molecule encoding a B2M mature protein.

78. The method according to any one of claims 64-77, wherein the modified cells have reduced allogeneic rejection response compared to cells without the modification.

79. The method according to any one of claims 65-78, wherein the presenting peptide is capable of forming a complex with the B2M protein and the heavy chain of the MHC molecule.

80. The method according to claim 79, wherein the complex is capable of inhibiting immune cell activation.

81. The method according to claim 80, wherein the immune cell is selected from the group comprising a natural killer (NK) cell, a T lymphocyte, a B lymphocyte, a NKT cell, a monocyte, a macrophage, a mast cell, a granulocyte, a dendritic cell, a lymphocyte, a leukocyte and/or a peripheral blood mononuclear cell.

82. The method according to any one of claims 80-81, wherein the immune cell is a natural killer (NK) cell, a NKT cell and/or a T lymphocyte.

83. The method according to any one of claims 80-82, wherein the inhibiting immune cell activation comprises inhibiting an immune cell from participating in an immune response.

84. The method according to any one of claims 80-83, wherein the inhibiting immune cell activation comprises binding to an inhibitory receptor of the immune cell.

85. The method according to claim 84, wherein the inhibitory receptor of the immune cell is selected from the group comprising NKG2A, NKG2B, KIR2DL1, KIR2DL2, KIR2DL3, KIR2DL4, KIR2DL5, KIR3DL1, KIR3DL2 and KIR3DL3.

86. The method according to claims 73-85, wherein the heavy chain of the MHC molecule comprises a heavy chain of a non-classical MHC class I molecule and/or a heavy chain of an MHC-like molecule.

87. The method according to claim 86, wherein the heavy chain of the non-classical MHC class I molecule comprises an HLA-E heavy chain, an HLA-F heavy chain and/or an HLA-G heavy chain.

88. The method according to any one of claims 86-87, wherein the heavy chain of the MHC-like molecule comprises a CD1 heavy chain, an MR1 heavy chain, an FcRn heavy chain and/or a UL18 heavy chain.

89. The method according to any one of claims 86-88, wherein the heavy chain of the MHC molecule is an HLA-E heavy chain.

90. The method according to any one of claims 64-89, wherein the presenting peptide is capable of binding to the peptide binding groove of the heavy chain of the MHC molecule.

91. The method according to any one of claims 64-90, wherein the presenting peptide is derived from a virus, a prokaryote, an eukaryote or a mammal.

92. The method according to any one of claims 64-91, wherein the presenting peptide is derived from a human.

93. The method according to any one of claims 64-92, wherein the presenting peptide comprises a non-classical MHC class I restricted presenting peptide and/or an MHC-like molecule restricted presenting peptide.

94. The method according to any one of claims 64-93, wherein the presenting peptide is a non-classical MHC class I restricted presenting peptide.

95. The method according to any one of claims 64-94, wherein the presenting peptide is an HLA-E restricted presenting peptide.

96. The method according to any one of claims 64-95, wherein the presenting peptide is derived from a protein selected from the group comprising an ATP-binding cassette transporter, a multidrug resistance-associated protein 7, gliadin, an HSP60 conserved region and a GroEL conserved region.

97. The method according to any one of claims 64-96, wherein the presenting peptide is derived from a signal peptide of a MHC class I molecule, or a fragment thereof.

98. The method according to any one of claims 64-97, wherein the presenting peptide is derived from a signal peptide of a protein selected from the group comprising HLA-Al, HLA-A2, HLA-A*02, HLA-A*0203, HLA-A*0210, HLA-A3, HLA-A*23, HLA-A*24, HLA-A*2403, HLA-A*2410, HLA-A*2502, HLA-A*2501, HLA-A*26, HLA-A*66(01,02), HLA-A9, HLA-A*6603, HLA-A10, HLA-A*11, HLA-A19, HLA-A*29, HLA-A*30, HLA-A*31, HLA-A*32, HLA-A*33, HLA-A*74, HLA-A*7403, HLA-A28, HLA-A36, HLA-A34, HLA-A43, HLA-A*3401, HLA-A*80, HLA-B5, HLA-B7, HLA-B8, HLA-B12, HLA-B13, HLA-B14, HLA-B15, HLA-B16, HLA-B17, HLA-B18, HLA-B21, HLA-B22, HLA-B27, HLA-B35, HLA-B37, HLA-B38, HLA-B39, HLA-B40, HLA-B41, HLA-B42, HLA-B46, HLA-B47, HLA-B48, HLA-B49, HLA-B50, HLA-B51, HLA-B52, HLA-B53, HLA-B54, HLA-B55, HLA-B56, HLA-B57, HLA-B58, HLA-B59, HLA-B60, HLA-B61, HLA-B62, HLA-B63, HLA-B64, HLA-B65, HLA-B67, HLA-B70, HLA-B71, HLA-B73, HLA-B75, HLA-B76, HLA-B77, HLA-B78, HLA-B81, HLA-B82, HLA-B83, HLA-Cw1, HLA-Cw2, HLA-Cw3, HLA-Cw4, HLA-Cw5, HLA-Cw6, HLA-Cw7, HLA-Cw8, HLA-Cw9, HLA-Cw10, HLA-Cw*0809, HLA-Cw7, HLA-Cw*1701, HLA-G and HLA-F.

99. The method according to any one of claims 64-98, wherein the presenting peptide is 5-30 amino acids in length.

100. The method according to any one of claims 64-99, wherein the presenting peptide is 7-20 amino acids in length.

101. The method according to any one of claims 64-90, wherein the presenting peptide is 8-10 amino acids in length.

102. The method according to any one of claims 64-101, wherein the presenting peptide comprises an amino acid sequence as shown in SEQ ID NO: 17.

103. The method according to any one of claims 62-102, wherein the presenting peptide comprises an amino acid sequence as shown in any one of SEQ ID NOs: 18-70.

104. The method according to any one of claims 62-103, wherein the presenting peptide comprises an amino acid sequence as shown in SEQ ID NO: 35, SEQ ID NO: 30 or SEQ ID NO: 33.

105. The method according to any one of claims 64-104, wherein the polynucleotide further comprises a nucleic acid molecule encoding a linker.

106. The method according to claim 105, wherein the presenting peptide and the B2M protein are linked via the linker in an expression product of the modified cell.

107. The method according to any one of claims 105-106, wherein the linker comprises a rigid linker and/or a flexible linker.

108. The method according to any one of claims 105-107, wherein the linker comprises an amino acid sequence as shown below: (EAAAK)ₙ, where n is 3, 4, or 5.

109. The method according to any one of claims 105-108, wherein the linker comprises an amino acid sequence as shown below: (GGGGS)ₙ, where n is 3, 4, or 5.

110. The method according to any one of claims 105-109, wherein the linker comprises an amino acid sequence as shown in any one of SEQ ID NOs: 71-99.

111. The method according to any one of claims 105-110, wherein the linker comprises an amino acid sequence as shown in SEQ ID NO: 99.

112. The method according to any one of claims 105-111, wherein the linker is located at the C-end of the presenting peptide.

113. The method according to any one of claims 64-112, wherein the polynucleotide does not comprise a nucleic acid molecule encoding a B2M protein or a fragment thereof.

114. The method according to any one of claims 64-113, wherein the polynucleotide encodes an amino acid sequence as shown in SEQ ID NO: 9.

115. The method according to any one of claims 64-114, wherein the polynucleotide comprises a nucleotide sequence as shown in SEQ ID NO: 12.

116. The method according to any one of claims 64-115, wherein the inserting is performed by introducing a vector comprising the polynucleotide.

117. The method according to claim 116, wherein the inserting comprises contacting a vector comprising the polynucleotide with the cell.

118. The method according to any one of claims 116-117, wherein the vector comprising the polynucleotide does not comprise a complete B2M gene coding region.

119. The method according to any one of claims 116-118, wherein the vector comprising the polynucleotide does not comprise a nucleic acid molecule encoding the heavy chain of the MHC molecule.

120. The method according to any one of claims 116-119, wherein the vector comprising the polynucleotide further comprises a homology arm.

121. The method according to any one of claims 116-120, wherein the vector comprising the polynucleotide comprises an upstream homology arm, the polynucleotide encoding the presenting peptide and a downstream homology arm.

122. The method according to any one of claims 116-121, wherein the vector comprising the polynucleotide comprises a nucleotide sequence as shown in SEQ ID NO: 4.

123. The method according to any one of claims 64-122, wherein the method further comprises introducing at least one sequence-specific agent that specifically targets the locus of the endogenous B2M gene into the cell.

124. The method according to claim 123, wherein the sequence-specific agent comprises a nuclease agent.

125. The method according to any one of claims 123-124, wherein the sequence-specific agent comprises a RNA and/or DNA-guided endonuclease.

126. The method according to claim 125, wherein the endonuclease comprises a Cas protein.

127. The method according to claim 126, wherein the Cas protein comprises a Cas9 protein.

128. The method according to any one of claims 123-127, wherein the sequence-specific agent further comprises a guide RNA (gRNA).

129. The method according to claim 128, wherein the gRNA is a single-stranded guide RNA.

130. The method according to any one of claims 128-129, wherein the gRNA comprises a nucleotide sequence as shown in SEQ ID NO: 13.

131. The method according to any one of claims 128-130, wherein the gRNA comprises 5'-(X)n-SEQ ID NO: 13-backbone sequence-3', where X is a base selected from any one of A, U, C and G, and n is any one integer of 0-15.

132. The method according to any one of claims 128-131, wherein the gRNA comprises a nucleotide sequence as shown in SEQ ID NO: 3.

133. The method according to any one of claims 64-132, wherein the cell comprises a mammalian cell.

134. The method according to any one of claims 64-133, wherein the cell comprises a stem cell and/or a somatic cell.

135. The method according to any one of claims 64-134, wherein the cell comprises a blood cell, a muscle cell, a nerve cell, and/or an immune cell.

136. The method according to any one of claims 64-135, wherein the cell comprises an immune cell selected from the group comprising a natural killer (NK) cell, a T lymphocyte, a B lymphocyte, a NKT cell, a monocyte, a macrophage, a mast cell, a granulocyte, a dendritic cell, a lymphocyte, a leukocyte and/or a peripheral blood mononuclear cell.

137. The method according to any one of claims 64-136, wherein the cell is a natural killer (NK) cell, a NKT cell and/or a T lymphocyte.

138. The method according to any one of claims 64-137, wherein the expression of a classical MHC class I molecule on the cell surface of the modified cell is down-regulated, and the expression of a non-classical MHC class I molecule is positive.

139. The method according to any one of claims 64-138, wherein the expression of a classical MHC class I molecule on the cell surface of the modified cell is down-regulated, and the expression of a non-classical MHC class I molecule is substantially not down-regulated.

140. The method according to any one of claims 64-139, wherein the expression of a classical MHC class I molecule on the cell surface of the modified cell is down-regulated, and the expression of a non-classical MHC class I molecule is up-regulated.

141. The method according to any one of claims 64-140, wherein the method further comprises down-regulating the expression and/or activity of a T cell receptor α constant region protein (TRAC), a T cell receptor β constant region protein (TRBC), CD3ε, CD3y, CD3δ and/or CD3ζ.

142. The method according to any one of claims 64-141, wherein the method further comprises administering a guide RNA targeting a nucleic acid molecule encoding the TRAC to the cell.

143. The method according to any one of claims 64-142, wherein the guide RNA targeting the nucleic acid molecule encoding the TRAC comprises an amino acid sequence as shown in SEQ ID NO: 8.

144. The method according to any one of claims 1-143, wherein the method further comprises allowing the modified cell to express a chimeric antigen receptor (CAR), a T cell receptor (TCR), a chimeric autoantibody receptor (CAAR), a TCR receptor fusion construct (TRuC), a T cell antigen coupler (TAC), an antibody TCR receptor (AbTCR), a chimeric CD3ε receptor and/or at least one other synthetic receptor.

145. The method according to any one of claims 1-144, wherein the method further comprises introducing a nucleic acid molecule encoding a chimeric antigen receptor (CAR), a T cell receptor (TCR), a chimeric autoantibody receptor (CAAR), a TCR receptor fusion construct (TRuC), a T cell antigen coupler (TAC), an antibody TCR receptor (AbTCR), a chimeric CD3ε receptor and/or at least one synthetic receptor into the modified cell.

146. A modified cell prepared by the method according to any one of claims 64-145.

147. An isolated nucleic acid molecule comprising the polynucleotide in the method according to any one of claims 1-63.

148. A guide RNA in the method according to any one of claims 128-145.

149. A nucleic acid combination comprising the isolated nucleic acid molecule according to claim 147 and the guide RNA according to claim 148.

150. A vector comprising the isolated nucleic acid molecule according to claim 147, the guide RNA according to claim 148 and/or the nucleic acid combination according to claim 149.

151. The vector according to claim 150, wherein the vector is a viral vector.

152. The vector according to any one of claims 150-151, wherein the vector is a lentiviral vector, an adenoviral vector or an adeno-associated virus vector.

153. A cell comprising the isolated nucleic acid molecule according to claim 147, the guide RNA according to claim 148, the nucleic acid combination according to claim 149, and/or the vector according to any one of claims 150-152.

154. A cell population comprising at least 30% of the modified cell according to any one of claims 1-63.

155. A pharmaceutical composition comprising the modified cell according to any one of claims 1-63 and 153, the isolated nucleic acid molecule according to claim 147, the guide RNA according to claim 148, the nucleic acid combination according to claim 149, the vector according to any one of claims 150-152, the cell according to claim 153 and/or the cell population according to claim 154, and optionally a pharmaceutically acceptable carrier.

156. Use of the modified cell according to any one of claims 1-63 and 146, the isolated nucleic acid molecule according to claim 147, the guide RNA according to claim 148, the nucleic acid combination according to claim 149, the vector according to any one of 150-152, the cell according to claim 153, the cell population according to claim 154 and/or the pharmaceutical composition according to claim 155 in the preparation of a medicine for allogeneic transplantation.

157. A method for allogeneic transplantation, wherein the method comprises administering the modified cell according to any one of claims 1-63 and 146, the isolated nucleic acid molecule according to claim 147, the guide RNA according to claim 148, the nucleic acid combination according to claim 149, the vector according to any one of 150-152, the cell according to claim 153, the cell population according to claim 154 and/or the pharmaceutical composition according to claim 155 to a subject.

158. A method for increasing allogeneic transplantation compatibility, wherein the method comprises using the method according to any one of claims 64-145.

159. Use of the modified cell according to any one of claims 1-63 and 146, the isolated nucleic acid molecule according to claim 147, the guide RNA according to claim 148, the nucleic acid combination according to claim 149, the vector according to any one of 150-152, the cell according to claim 153, the cell population according to claim 154 and/or the pharmaceutical composition according to claim 155 in the preparation of a medicine for the treatment or alleviation of a tumor.

160. A method for treating or alleviating a tumor, wherein the method comprises administering the modified cell according to any one of claims 1-63 and 146, the isolated nucleic acid molecule according to claim 147, the guide RNA according to claim 148, the nucleic acid combination according to claim 149, the vector according to any one of 150-152, the cell according to claim 153, the cell population according to claim 154 and/or the pharmaceutical composition according to claim 155 to a subject.
